(19) Europäisches Patentamt
European Patent Office
Office européen des brevets

(11) Veröffentlichungsnummer: **0 457 105 B1**

(12) **EUROPÄISCHE PATENTSCHRIFT**

(45) Veröffentlichungstag der Patentschrift: **26.07.95**

(51) Int. Cl.$^6$: **C07D 319/06**, C09K 19/58

(21) Anmeldenummer: **91107070.4**

(22) Anmeldetag: **02.05.91**

# (54) Chirale Dioxane als Komponenten flüssigkristalliner Gemische.

(30) Priorität: **15.05.90 CH 1635/90**

(43) Veröffentlichungstag der Anmeldung:
**21.11.91 Patentblatt 91/47**

(45) Bekanntmachung des Hinweises auf die Patenterteilung:
**26.07.95 Patentblatt 95/30**

(84) Benannte Vertragsstaaten:
**CH DE FR GB IT LI NL**

(56) Entgegenhaltungen:
**EP-A- 0 182 054**

(73) Patentinhaber: **F. HOFFMANN-LA ROCHE AG**
**Postfach 3255**
**CH-4002 Basel (CH)**

(72) Erfinder: **Buchecker, Richard, Dr.**
**Felsenstrasse 10**
**CH-8008 Zürich (CH)**
Erfinder: **Fünfschilling, Jürg, Dr.**
**Weierhofstrasse 138**
**CH-4045 Basel (CH)**
Erfinder: **Schadt, Martin, Dr.**
**Liestalerstrasse 77**
**CH-4411 Seltisberg (CH)**

(74) Vertreter: **Cottong, Norbert A. et al**
**Grenzacherstrasse 124**
**Postfach 3255**
**CH-4002 Basel (CH)**

**EP 0 457 105 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue chirale Dotierstoffe für Flüssigkristalle sowie flüssigkristalline Gemische, die solche Dotierstoffe enthalten, und deren Verwendung für optische und elektro-optische Zwecke.

Flüssigkristallmaterialien für elektro-optische Anzeigevorrichtungen enthalten häufig einen oder mehrere optisch aktive Zusatzstoffe zur Induzierung einer chiralen Struktur. Beispielsweise wird zur Verwendung in Anzeigevorrichtungen mit verdrillt nematischer Struktur bevorzugt ein nematischer Flüssigkristall mit optisch aktivem Zusatzstoff dotiert, z.B. um eine Umkehr der Verdrillungsrichtung (reverse twist) in TN-Zellen (twisted-nematic) zu vermeiden oder um eine ausreichende Verdrillung in Zellen mit hoch verdrillter nematischer Struktur, wie STN-Zellen (super twisted-nematic), SBE-Zellen (super birefringence effect) oder OMI-Zellen (optical mode interference) zu erreichen. Ferner können cholesterische Flüssigkristalle für Phase-Change-Zellen vorzugsweise aus einem nematischen Basismaterial und einem oder mehreren optisch aktiven Dotierstoffen bestehen und ferroelektrische Flüssigkristalle für Anzeigevorrichtungen auf der Basis von chiral getilteten smektischen Phasen können vorzugsweise aus einem Material mit getilteter smektischer Phase und einem oder mehreren optisch aktiven Dotierstoffen bestehen.

Die elektro-optischen Kennlinien von Flüssigkristall-Anzeigevorrichtungen sind temperaturabhängig, was insbesondere beim Multiplexbetrieb störend ist. Es ist jedoch bekannt, dass diese Temperaturabhängigkeit mindestens teilweise kompensiert werden kann durch Zusatz von chiralem Dotierstoff, welcher eine mit steigender Temperatur abnehmende Ganghöhe induziert. Eine solche inverse Temperaturabhängigkeit wurde nur für wenige Verbindungen gefunden. Sie kann aber auch durch Verwendung von mindestens 2 chiralen Dotierstoffen erreicht werden, welche eine unterschiedliche relative Temperaturabhängigkeit aufweisen und unterschiedliche Verdrillungsrichtung induzieren (DE-A-2827471). Allerdings erfordert dies meist einen relativ hohen Anteil an chiralen Dotierstoffen.

Cholesterische Flüssigkristalle reflektieren Licht in einem Wellenlängenbereich, für den die Wellenlänge etwa gleich der Helixganghöhe ist. Die spektrale Breite dieses Reflexionslichtes kann durch geeignete Wahl des Flüssigkristalles variiert werden. Das reflektierte Licht ist vollständig zirkular polarisiert. Die Drehrichtung des reflektierten Lichts hängt vom Drehsinn der cholesterischen Helixstruktur ab. Das entgegengesetzt zirkular polarisierte Licht wird ungeschwächt transmittiert. Diese Eigenschaften können zur Herstellung von optischen Filtern, Polarisatoren, Analysatoren etc. ausgenutzt werden. Ferner wurden cholesterische Flüssigkristalle auch verschiedentlich für thermochrome Anwendungen und in kosmetischen Präparaten verwendet.

Cholesterische Flüssigkristalle für obige Anwendungen können vorzugsweise aus einem nematischen oder cholesterischen Basismaterial und einem oder mehreren chiralen Dotierstoffen bestehen, was eine einfache Einstellung der gewünschten Helixganghöhe erlaubt.

Um cholesterische Gemische mit einer Ganghöhe im Bereich der Wellenlänge des sichtbaren Lichts zu erreichen, sollten die chiralen Dotierstoffe ein hohes Verdrillungsvermögen aufweisen und in üblichen Flüssigkristallmaterialien gut löslich sein. Weiterhin sollten die chiralen Dotierstoffe eine ausreichende Stabilität besitzen, mit dem Mesophasentyp des Flüssigkristallmaterials gut kompatibel sein und den Mesophasenbereich nicht zu stark einschränken. Solche Eigenschaften wären auch für chirale Dotierstoffe zur Erzielung der eingangs genannten, verdrillt nematischen Strukturen erwünscht, da ihr Anteil vergleichsweise niedrig gehalten werden könnte und somit die Eigenschaften des Flüssigkristallmaterials nur unwesentlich beeinflusst würden.

Die Verwendung chiraler Dotierstoffe in ferroelektrischen Flüssigkristallen dient vor allem zur Erzeugung oder Veränderung einer Verdrillung der getilteten smektischen Phase und zur Induzierung einer spontanen Polarisation. Die chiralen Dotierstoffe sollten eine ausreichende Stabilität besitzen, mit dem getilteten smektischen Flüssigkristall gut kompatibel sein und den Mesophasenbereich nicht zu stark einschränken. Ferner wäre es wünschenswert, dass die Verdrillung und die spontane Polarisation möglichst unabhängig voneinander eingestellt werden können und die Viskosität der chiralen Dotierstoffe vergleichsweise niedrig ist. Dem Fachmann sind zahlreiche Dotierstoffe bekannt. So werden beispielsweise in EP-A-0 182 054 flüssigkristalline Derivate von in 2-, 4- oder 5-Stellung substituierten Dioxan-Ringen beschrieben, welche als chirale Dotierstoffe verwendet werden können, wenn sie einen optisch aktiven Rest tragen.

Die Erfindung betrifft optisch aktive 4-Methyl-1,3-dioxan-Derivate der allgemeinen Formel

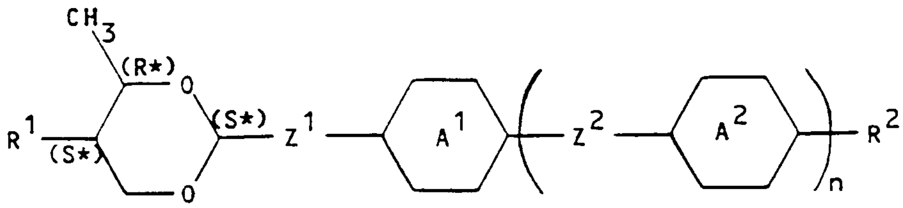

I

worin n für die Zahl 0 oder 1 steht; $Z^1$ eine einfache Kovalenzbindung oder $-CH_2CH_2-$ bezeichnet; $Z^2$ eine einfache Kovalenzbindung, $-CH_2CH_2-$, $-CH_2O-$, $-OCH_2-$, -COO- oder -OOC- bezeichnet; die Ringe $A^1$ und $A^2$ unabhängig voneinander trans-1,4-Cyclohexylen oder gegebenenfalls mit Halogen, Cyano und/oder Methyl substituiertes 1,4-Phenylen, in welchem gegebenenfalls 1 oder 2 CH-Gruppen durch Stickstoff ersetzt sind, darstellen; $R^2$ eine Gruppe $R^4$ oder eine Gruppe der allgemeinen Formel

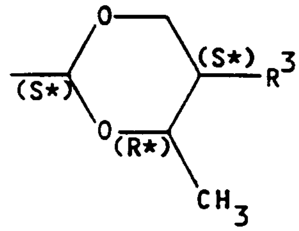

II

bezeichnet; $R^4$ Cyano, Halogen, $-OCY^1F_2$, $-CY^1F_2$ oder eine Alkylgruppe mit 1-18 Kohlenstoffatomen bedeutet, in welcher gegebenenfalls eine >CH-CH< durch >C = C< ersetzt ist und/oder gegebenenfalls eine oder zwei nicht benachbarte Methylengruppen durch -O-, -COO- und/oder -OOC- ersetzt sind und/oder gegebenenfalls eine Methylengruppe durch $-CHY^2-$ ersetzt ist; $Y^1$ Wasserstoff oder Fluor bezeichnet; $Y^2$ Halogen, Cyano oder Methyl bedeutet; $R^1$ und $R^3$ Alkyl mit 1-18 Kohlenstoffatomen bezeichnen, in welchem gegebenenfalls eine >CH-CH< durch >C = C< ersetzt ist und/oder gegebenenfalls eine oder zwei nicht benachbarte Methylengruppen durch -O- ersetzt sind und/oder gegebenenfalls ein oder mehrere Wasserstoffatome durch Fluor ersetzt sind; und (S*) und (R*) die relativen Konfigurationen der chiralen Kohlenstoffatome bezeichnen.

Die erfindungsgemässen Verbindungen sind farblos, in üblichen Flüssigkristallen gut löslich und insbesondere gut kompatibel mit nematischen, cholesterischen und getilteten smektischen Phasen. Sie sind ausreichend stabil gegen elektrische und magnetische Felder und besitzen häufig selbst flüssigkristalline Eigenschaften. Obwohl der chirale Dioxanring relativ unpolar ist, induzieren sie in getilteten smektischen Phasen eine vergleichsweise hohe spontane Polarisation. Ferner besitzen sie ein hohes Verdrillungsvermögen und sind vergleichsweise niederviskos. Verdrillungsvermögen und spontane Polarisation können gewünschtenfalls durch chirale Endgruppen weiter verstärkt oder modifiziert werden. Ferner kann durch laterale Substitution u.a. die spontane Polarisation relativ zum Verdrillungsvermögen verändert werden; beispielsweise kann durch laterale Substitution (z.B. mit Halogen) an einem Benzolring $A^1$ in ortho-Stellung zum Dioxanring bei gleicher Helizität das Vorzeichen der spontanen Polarisation umgekehrt werden. Dies ermöglicht eine weitgehend voneinander unabhängig Variierung der Verdrillung und der spontanen Polarisation.

Der Ausdruck "Halogen" bezeichnet im Rahmen der vorliegenden Erfindung Fluor, Chlor, Brom und Jod, insbesondere Fluor, Chlor und Brom.

Der Ausdruck "gegebenenfalls mit Halogen, Cyano und/oder Methyl substituiertes 1,4-Phenylen, in welchem gegebenenfalls 1 oder 2 CH-Gruppen durch Stickstoff ersetzt sind", umfasst Gruppen wie 1,4-Phenylen, 2-Fluor-1,4-phenylen, 2-Chlor-1,4-phenylen, 2-Brom-1,4-phenylen, 2-Cyano-1,4-phenylen, 2-Methyl-1,4-phenylen, 2,3-Difluor-1,4-phenylen, 2,3-Dicyano-1,4-phenylen, Pyridin-2,5-diyl, Pyrimidin-2,5-diyl, Pyrazin-2,5-diyl, Pyridazin-3,6-diyl und dergleichen.

Der Ausdruck "Alkylgruppe, in welcher gegebenenfalls eine >CH-CH< durch >C = C< ersetzt ist und/oder gegebenenfalls eine oder zwei nicht benachbarte Methylengruppen durch -O-, -COO- und/oder -OOC- ersetzt sind und/oder gegebenenfalls eine Methylengruppe durch $-CHX^2-$ ersetzt ist", umfasst geradkettige und verzweigte (gegebenenfalls chirale) Reste wie Alkyl, 1E-Alkenyl, 3E-Alkenyl, 4-Alkenyl,

Alkenyl mit endständiger Doppelbindung, Alkoxy, 2E-Alkenyloxy, 3-Alkenyloxy, Alkenyloxy mit endständiger Doppelbindung, Alkoxyalkyl, Alkenyloxyalkyl, Alkoxycarbonyl, Alkoxycarbonylalkoxy, Alkoxycarbonylalkoxycarbonyl, Alkanoyloxy, 1-Haloalkyl, 2-Haloalkyl, 2-Haloalkoxy, 2-Haloalkanoyloxy, 1-Cyanoalkyl, 2-Cyanoalkyl, 2-Cyanoalkoxy, 1-Methylalkyl, 2-Methylalkyl, 1-Methylalkoxy, 2-Methylalkoxy und dergleichen. Beispiele bevorzugter Reste sind Methyl, Aethyl, Propyl, Butyl, Pentyl, Hexyl, Heptyl, Octyl, Nonyl, Decyl, Undecyl, Dodecyl, 1-Methylpropyl, 1-Methylheptyl, 2-Methylbutyl, Vinyl, 1E-Propenyl, 1E-Butenyl, 1E-Pentenyl, 3-Butenyl, 3E-Pentenyl, 3E-Hexenyl, 3E-Heptenyl, 4-Pentenyl, 4Z-Hexenyl, 5-Hexenyl, 6-Heptenyl, 7-Octenyl, Methoxy, Aethoxy, Propyloxy, Butyloxy, Pentyloxy, Hexyloxy, Heptyloxy, Octyloxy, Nonyloxy, Decyloxy, Undecyloxy, Dodecyloxy, 1-Methylpropyloxy, 1-Methylheptyloxy, 2-Methylbutyloxy, 3-Methylpentyloxy, Allyloxy, 2E-Butenyloxy, 2E-Pentenyloxy, 2E-Hexenyloxy, 3-Butenyloxy, 3Z-Pentenyloxy, 4-Pentenyloxy, 5-Hexenyloxy, 6-Heptenyloxy, 7-Octenyloxy, Methoxymethyl, Aethoxymethyl, Propyloxymethyl, Allyloxymethyl, Methoxycarbonyl, Aethoxycarbonyl, Propyloxycarbonyl, 1-Methylpropyloxycarbonyl (= 2-Butyloxycarbonyl), 1-Methylpentyloxycarbonyl (= 2-Hexyloxycarbonyl), 1-Methylheptyloxycarbonyl (= 2-Octyloxycarbonyl), 1-(Methoxycarbonyl)äthoxy, 1-(Aethoxycarbonyl)äthoxy, 1-(Methoxycarbonyl)äthoxycarbonyl, 1-(Isobutyloxycarbonyl)äthoxycarbonyl, Acetoxy, Propionyloxy, Butyryloxy, 2-Fluorhexanoyloxy, 2-Fluorpentyloxycarbonyl, 2-Fluor-3-methylbutyloxycarbonyl, 2-Fluor-4-methylpentyloxycarbonyl und dergleichen.

Der Ausdruck "Alkyl, in welchem gegebenenfalls eine >CH-CH< durch >C=C< ersetzt ist und/oder gegebenenfalls eine oder zwei nicht benachbarte Methylengruppen durch -O- ersetzt sind und/oder gegebenenfalls ein oder mehrere Wasserstoffatome durch Fluor ersetzt sind", umfasst Alkyl, Alkenyl (z.B. 3E-Alkenyl, 4-Alkenyl, Alkenyl mit endständiger Doppelbindung), Alkoxyalkyl (z.B. 2-Alkoxyäthyl, 3-Alkoxypropyl), Fluoralkyl und dergleichen. Die Reste können geradkettig oder verzweigt (gegebenenfalls chiral) sein. Beispiele bevorzugter Reste sind Methyl, Aethyl, Propyl, Butyl, Pentyl, Hexyl, Heptyl, Octyl, Nonyl, Decyl, Undecyl, Dodecyl, 1-Methylpropyl, 1-Methylheptyl, 2-Methylbutyl, 3-Butenyl, 3E-Pentenyl, 3E-Hexenyl, 3E-Heptenyl, 4-Pentenyl, 4Z-Hexenyl, 5-Hexenyl, 6-Heptenyl, 7-Octenyl, 2-Methoxyäthyl, 3-Methoxypropyl, 1-Fluorpropyl, 2-Fluorpentyl und dergleichen.

Die in Formel I und den folgenden Formeln mit (S*) und (R*) bezeichneten relativen Konfigurationen der chiralen Kohlenstoffatome bedeuten, dass die bezeichneten Kohlenstoffatome wie angegeben die (S)- oder (R)-Konfiguration aufweisen oder, dass alle bezeichneten Kohlenstoffatome die spiegelbildliche Konfiguration aufweisen. Die Formeln umfassen somit jeweils das Diastereoisomer, worin (S*) für die (S)-Konfiguration und (R*) für die (R)-Konfiguration steht, sowie dessen optischen Antipoden, worin (S*) für die (R)-Konfiguration und (R*) für die (S)-Konfiguration steht.

Ring $A^2$ in obiger Formel I kann vorzugsweise 1,4-Phenylen oder mit Halogen substituiertes 1,4-Phenylen bedeuten. Bevorzugte halogen-substituierte Ringe sind 2-Fluor-1,4-phenylen und 2,3-Difluor-1,4-phenylen.

$Z^2$ kann vorzugsweise eine einfache Kovalenzbindung, $-CH_2CH_2-$ oder -OOC-, insbesondere eine einfache Kovalenzbindung bezeichnen. $Z^1$ kann vorzugsweise eine einfache Kovalenzbindung bezeichnen.

Besonders bevorzugt sind somit im allgemeinen diejenigen Verbindungen der Formel I, worin Ring $A^2$ 1,4-Phenylen, 2-Fluor-1,4-phenylen oder 2,3-Difluor-1,4-phenylen darstellt und $Z^1$ und $Z^2$ einfache Kovalenzbindungen bezeichnen.

Ring $A^1$ kann vorzugsweise Pyridin-2,5-diyl, Pyrimidin-2,5-diyl, trans-1,4-Cyclohexylen oder gegebenenfalls mit Halogen, Cyano und/oder Methyl substituiertes 1,4-Phenylen darstellen. Besonders bevorzugte Bedeutungen von Ring $A^1$ sind trans-1,4-Cyclohexylen, 1,4-Phenylen und mit Halogen substituiertes 1,4-Phenylen, insbesondere 1,4-Phenylen, 2-Fluor-1,4-phenylen, 2,3-Difluor-1,4-phenylen und 2-Chlor-1,4-phenylen.

Bevorzugte Untergruppen von Verbindungen der Formel I sind die Verbindungen der allgemeinen Formeln

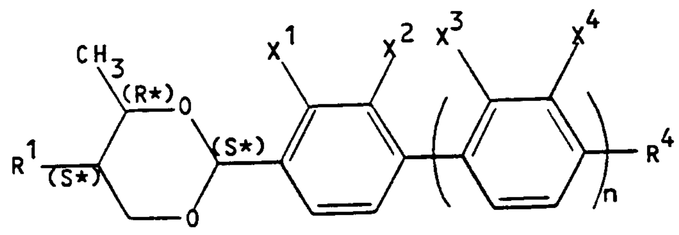

I-1

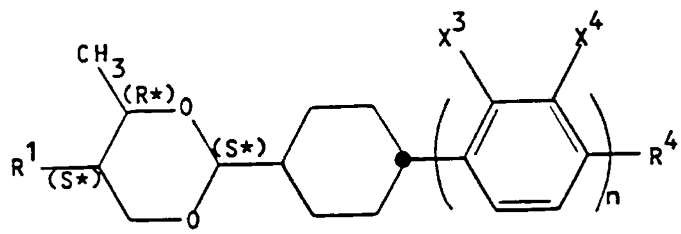

I-2

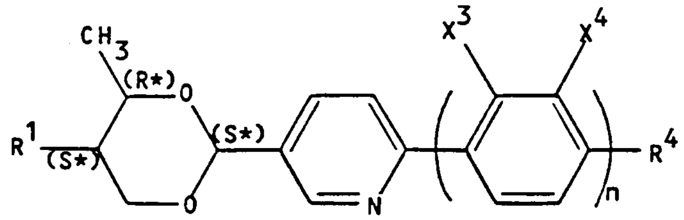

I-3

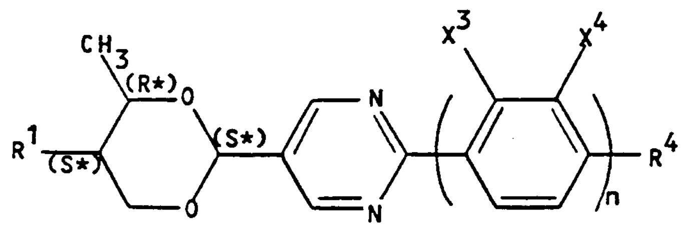

I-4

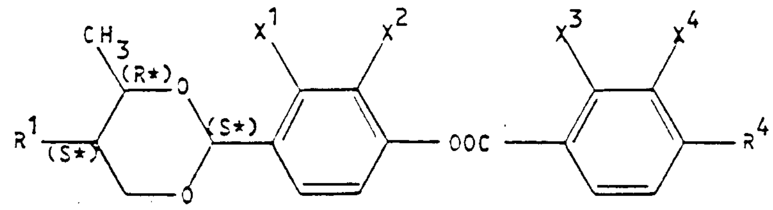

I-5

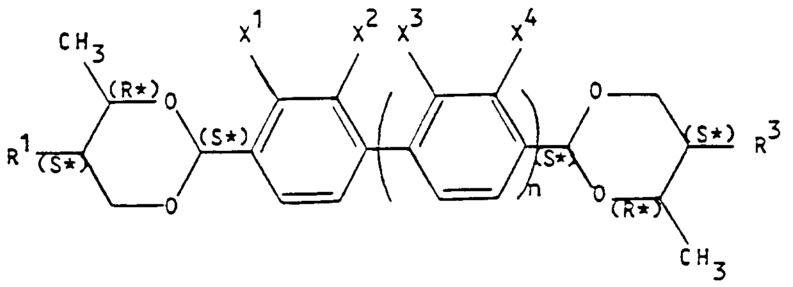

I-6

worin $R^4$ Cyano,Halogen, $-OCY^1F_2$, $-CY^1F_2$ oder eine Alkylgruppe bedeutet, in welcher gegebenenfalls eine >CH-CH< durch >C=C< ersetzt ist und/oder gegebenenfalls eine oder zwei nicht benachbarte Methylengruppen durch -O-, -COO- und/oder -OOC- esetzt sind und/oder gegebenenfalls eine Methylengruppe durch $-CHY^2-$ ersetzt ist; $Y^1$ Wasserstoff oder Fluor bezeichnet; $Y^2$ Halogen, Cyano oder Methyl bedeutet; $X^1$, $X^2$, $X^3$ und $X^4$ unabhängig voneinander Wasserstoff oder Halogen bezeichnen; und n, $R^1$, $R^3$, (S*) und (R*) die obigen Bedeutungen haben.

Die Reste $R^1$, $R^3$ und $R^4$ in den obigen Formeln I und I-1 bis I-6 können unabhängig voneinander vorzugsweise 1 bis etwa 18 Kohlenstoffatome aufweisen. Besonders bevorzugt sind im allgemeinen Reste mit 1 bis 12 Kohlenstoffatomen.

Die Reste $R^1$ und $R^3$ bedeuten vorzugsweise Alkyl, insbesondere geradkettiges Alkyl. Gewünschtenfalls können die Reste $R^1$ und $R^3$ aber eine Doppelbindung, 1-2 Sauerstoffatome, ein oder mehrere Fluoratome und/oder Kettenverzweigungen aufweisen, beispielsweise wenn eine geringfügige Modifizierung der Eigenschaften oder wenn chirale Reste erwünscht sind. $R^1$ und $R^3$ können insbesondere auch identische Reste bedeuten.

Bevorzugte Reste $R^4$ in den obigen Formeln I und I-1 bis I-5 sind Cyano, Fluor, Chlor, Difluormethoxy, Trifluormethoxy, Difluormethyl, Trifluormethyl, Alkyl, Alkoxy, Alkoxycarbonyl, 1-(Alkoxycarbonyl)-äthoxycarbonyl, Alkanoyloxy, 2-Fluoralkanoyloxy, Alkenyl und Alkenyloxy.

Bevorzugte Reste $R^2$ sind die für $R^4$ als bevorzugt genannten Reste sowie die Gruppen der Formel II, worin $R^3$ die oben als bevorzugt genannten Bedeutungen besitzt.

Die Substituenten $X^1$, $X^2$, $X^3$ und $X^4$ in den obigen Formeln I-1 bis I-6 können unabhängig voneinander vorzugsweise Wasserstoff, Fluor oder Chlor bedeuten. Vorzugsweise sind jeweils höchstens einer oder zwei der Substituenten von Wasserstoff verschieden. Besonders bevorzugt sind im allgemeinen diejenigen Verbindungen der Formeln I-1 bis I-6, worin $X^1$ Wasserstoff, Fluor oder Chlor bezeichnet, $X^2$ und $X^3$ Wasserstoff bezeichnen und $X^4$ Wasserstoff, Fluor oder Chlor bezeichnet.

Die Verbindungen der Formel I können dadurch hergestellt werden, dass man ein Diol der allgemeinen Formel

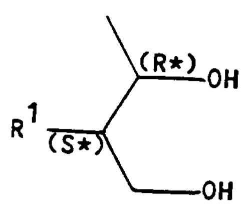

III

mit einem Aldehyd der allgemeinen Formel

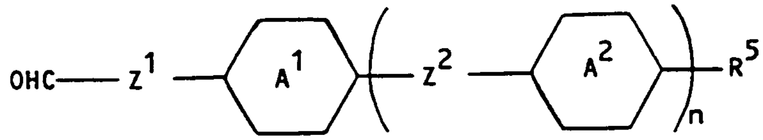

IV

worin $R^5$ eine Gruppe $R^2$ oder Formyl bezeichnet und $R^1$, $R^2$, $Z^1$, $Z^2$, n, (R*), (S*) und die Ringe $A^1$ und $A^2$ die obigen Bedeutungen haben,
oder einem Acetal hiervon umsetzt.

Die Umsetzung des Aldehyds der Formel IV oder eines geeigneten Acetals (z.B. Dimethylacetal) mit dem Diol der Formel III kann in an sich bekannter Weise erfolgen. Vorzugsweise erfolgt die Umsetzung in einem inerten organischen Lösungsmittel (beispielsweise einem aromatischen Kohlenwasserstoff, wie Benzol, Toluol oder Xylol) in Gegenwart einer katalytischen Menge einer organischen oder anorganischen Säure, wie p-Toluolsulfonsäure, Schwefelsäure oder trockenem Chlorwasserstoff. Temperatur und Druck sind nicht kritisch. Vorzugsweise wird jedoch bei Atmosphärendruck und Rückflusstemperatur unter Abtrennung des gebildeten Wassers gearbeitet.

Wenn $R^2$ in Formel I eine Gruppe der Formel II bedeutet, kann die Bildung der beiden Dioxanringe wie oben angedeutet nacheinander oder - insbesondere wenn $R^1$ und $R^3$ identisch sind - auch gleichzeitig erfolgen.

Wenn $Z^2$ und/oder $R^2$ Estergruppen aufweisen, kann vorzugsweise die Veresterung erst nach der Bildung des Dioxanringes erfolgen. Ferner kann, wenn $R^1$, $Z^2$ und/oder $R^2$ Aethergruppen aufweisen, gewünschtenfalls die Verätherung auch nach der Bildung des Dioxanringes erfolgen.

Die Aldehyde der Formel IV sind bekannte oder Analoge bekannter Verbindungen und können nach bekannten Methoden hergestellt werden.

Die Herstellung der Diole der Formel III kann in an sich bekannter Weise erfolgen. Eine bevorzugte Methode ist die Einführung des Substituenten $R^1$ in 2-Stellung von 3-Hydroxybuttersäure-alkylester - z.B. durch Umsetzung mit dem entsprechenden Alkylhalogenid, Alkenylhalogenid etc. in Gegenwart einer geeigneten Base in analoger Weise zu Tetrahedron 40, 1269 (1984) - und anschliessende Reduktion der Estergruppe. Beispielsweise kann 3-Hydroxybuttersäure-äthylester mit Alkylbromid, Alkenylbromid und dergleichen in Gegenwart einer Base wie Lithiumdiisopropylamid umgesetzt und der erhaltene 3-Hydroxy-2$R^1$-buttersäure-äthylester mit Lithiumaluminiumhydrid reduziert werden.

Die erfindungsgemässen Verbindungen eignen sich als chirale Dotierstoffe für flüssigkristalline Gemische. Die Erfindung betrifft daher ebenfalls ein flüssigkristallines Gemisch mit mindestens 2 Komponenten, welches dadurch gekennzeichnet ist, dass mindestens eine Komponente eine optisch aktive Verbindung der Formel I ist.

Vorzugsweise enthalten die erfindungsgemässen Gemische ein flüssigkristallines Trägermaterial und eine oder mehrere optisch aktive Verbindungen der Formel I. Als Trägermaterial eignen sich grundsätzlich alle Flüssigkristallmaterialien, welche eine verdrillbare Flüssigkristallphase mit einem adäquaten Mesophasenbereich aufweisen. Das flüssigkristalline Trägermaterial kann eine Einzelverbindung oder ein Gemisch sein und besitzt vorzugsweise einen Klärpunkt von mindestens etwa 60°C. Besonders geeignet sind die Verbindungen der Formel I als chirale Dotierstoffe für Trägermaterialien mit nematischer, cholesterischer oder getilteter smektischer (vorzugsweise smektisch C) Phase.

Der Anteil an chiralem Dotierstoff der Formel I ist weitgehend durch das Verdrillungsvermögen, die spontane Polarisation und die gewünschte Ganghöhe bestimmt. Der Anteil an chiralem Dotierstoff kann daher je nach Anwendung in einem breiten Bereich variieren und beispielsweise etwa 0,1-40 Gew.-% betragen. Für Anzeigevorrichtungen auf der Basis von Flüssigkristallen mit verdrillt nematischer Struktur ist je nach Zellentyp und -dicke meist eine Ganghöhe von etwa 3-40 µm erforderlich und daher ein entsprechend kleiner Anteil, beispielsweise etwa 0,1-3 Gew.-%, ausreichend. Demgegenüber werden für Anwendungen, welche auf der Reflexion von sichtbarem Licht durch cholesterische Schichten beruhen, Ganghöhen von weniger als 2 µm, beispielsweise etwa 0,4-0,6 µm benötigt, was einen entsprechend höheren Anteil an chiralem Dotierstoff erfordert. Für solche Anwendungen und bei Verwendung in getilteten smektischen Phasen ist im allgemeinen ein Anteil von etwa 3-30 Gew.-%, insbesondere etwa 5-20 Gew.-% an optisch aktiven Dotierstoffen der Formel I bevorzugt.

Die erfindungsgemässen Gemische mit cholesterischer Phase können neben einer oder mehreren optisch aktiven Verbindungen der Formel I vorzugsweise eine oder mehrere Verbindungen der folgenden allgemeinen Formeln als Komponenten des nematischen oder cholesterischen Trägermaterials enthalten:

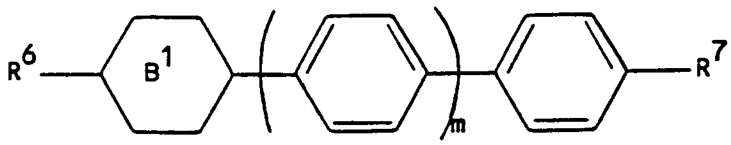

V

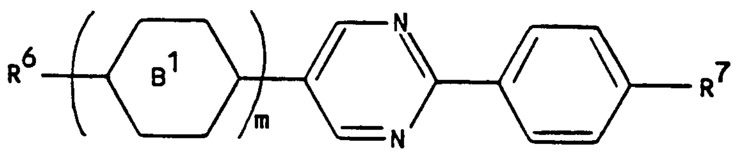

VI

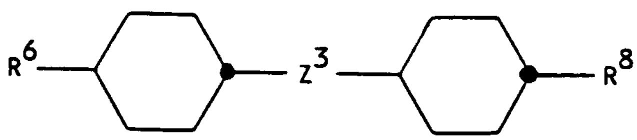

VII

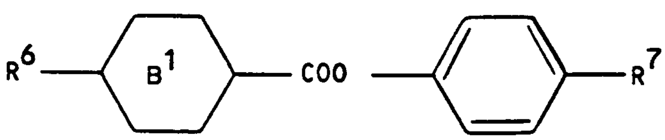

VIII

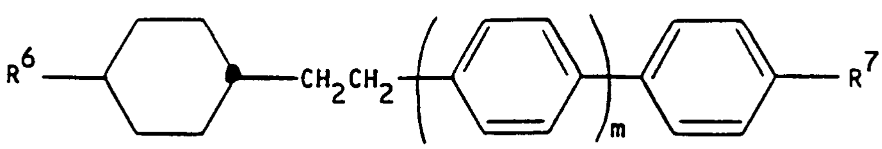

IX

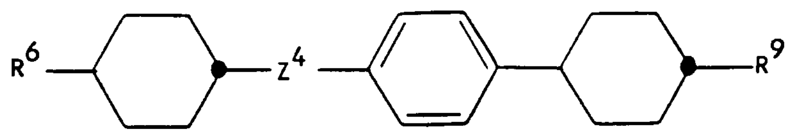

X

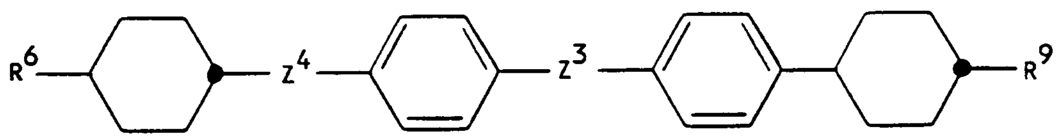

XI

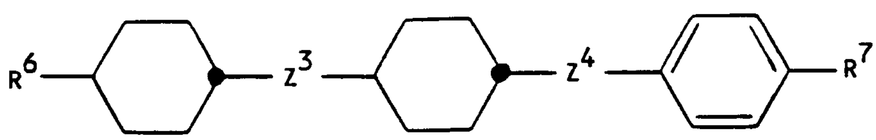

XII

worin m für die Zahl 0 oder 1 steht; Ring $B^1$ 1,4-Phenylen oder trans-1,4-Cyclohexylen darstellt; $R^6$ Alkyl, Alkenyl oder an einem Benzolring auch Alkoxy oder Alkenyloxy bezeichnet; $R^7$ Cyano, Alkyl, Alkoxy, Alkenyl oder Alkenyloxy bedeutet; $Z^3$ eine einfache Kovalenzbindung oder $-CH_2CH_2-$ bezeichnet; $R^8$ Cyano, Alkyl, Alkoxy, Alkenyl, Alkenyloxy, Alkoxymethyl oder Alkenyloxymethyl bedeutet; $Z^4$ eine einfache Kovalenzbindung, -COO- oder $-CH_2CH_2-$ bezeichnet; und $R^9$ Alkyl oder Alkenyl bedeutet.

$R^6$, $R^7$, $R^8$ und $R^9$ besitzen vorzugsweise höchstens je 12 Kohlenstoffatome, besonders bevorzugt höchstens je 7 Kohlenstoffatome. Bevorzugte Alkenylgruppen sind 3E-Alkenyl, 4-Alkenyl oder an einem Cyclohexan-Ring auch 1E-Alkenyl. Bevorzugte Alkenyloxygruppen sind 2E-Alkenyloxy und 3-Alkenyloxy.

Die erfindungsgemässen Gemische mit chiral getilteter smektischer Phase können neben einer oder mehreren optisch aktiven Verbindungen der Formel I vorzugsweise eine oder mehrere Verbindungen der folgenden allgemeinen Formeln als Komponenten des Trägermaterials enthalten:

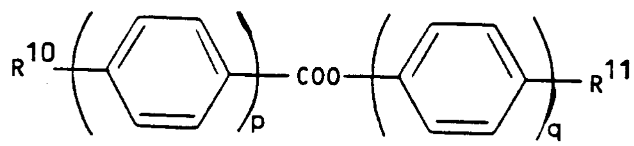

XIII

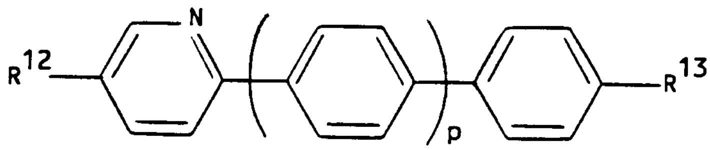

XIV

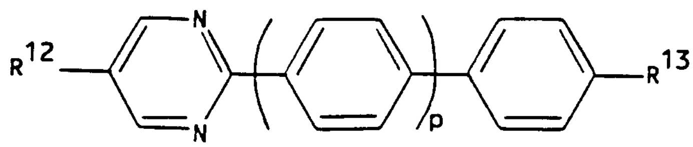

XV

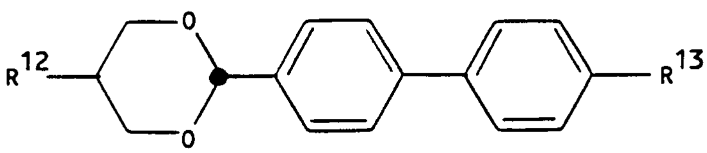

XVI

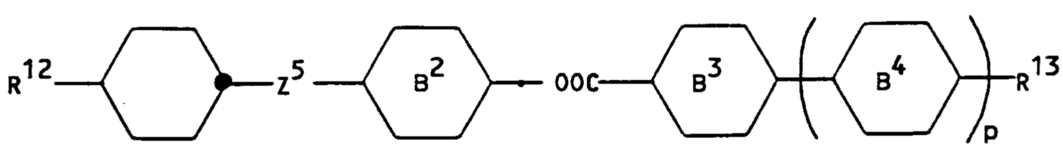

XVII

worin p und q unabhängig voneinander für die Zahl 0 oder 1 stehen; $R^{10}$ und $R^{11}$ unabhängig voneinander Alkyl, Alkoxy, Alkanoyloxy, Alkoxycarbonyl, Alkenyl oder Alkenyloxy bedeuten; $R^{12}$ Alkyl oder Alkenyl bezeichnet; $R^{13}$ Alkyl, Alkoxy, Alkenyl oder Alkenyloxy bedeutet; die Ringe $B^2$, $B^3$ und $B^4$ unabhängig voneinander 1,4-Phenylen oder halogen-substituiertes 1,4-Phenylen darstellen; und $Z^5$ eine einfache Kovalenzbindung, $-CH_2CH_2-$, $-CH_2O-$, -COO- oder -OOC- bezeichnet.

$R^{10}$, $R^{11}$, $R^{12}$ und $R^{13}$ besitzen vorzugsweise höchstens je 18 Kohlenstoffatome, besonders bevorzugt etwa 5-12 Kohlenstoffatome. Bevorzugte halogen-substituierte 1,4-Phenylengruppen sind 2-Fluor-1,4-phenylen und 2,3-Difluor-1,4-phenylen. Im allgemeinen sind diejenigen Verbindungen der Formeln XIII-XVII bevorzugt, worin $R^{11}$ bzw. $R^{13}$ Alkoxy oder Alkenyloxy bedeutet.

Die Herstellung der flüssigkristallinen Gemische und der elektro-optischen Vorrichtungen kann in an sich bekannter Weise erfolgen.

Die Erfindung wird durch die folgenden Beispiele weiter veranschaulicht. Optische Antipoden der Verbindungen der Formel I besitzen jeweils die gleichen Phasenumwandlungstemperaturen und induzieren die gleichen Absolutwerte der spontanen Polarisation und der Verdrillung aber mit entgegengesetztem Vorzeichen. Die zur Charakterisierung der Phasenumwandlungen verwendeten Abkürzungen stehen für folgende Bedeutungen:

| | |
|---|---|
| C | für kristallin, |
| S | für smektisch, |
| $S_A$, $S_B$, $S_C$ etc. | für smektisch A, B, C etc., |
| $S_C$*, $S_F$* etc. | für chiral smektisch C, F etc., |
| N | für nematisch, |
| N* | für cholesterisch, |
| I | für isotrop. |

Beispiel 1

In einem Rundkolben mit Magnetrührer, Wasserabscheider und Kühler wurde unter Stickstoff ein Gemisch von 3,0 g (2S,3R)-2-Octyl-1,3-butandiol, 3,07 g 4'-Cyano-4-biphenylcarboxaldehyd,0,1 ml 1N Schwefelsäure und 30 ml Toluol vorgelegt. Das Gemisch wurde unter Wasserabscheidung 3 Stunden zum Rückfluss erhitzt. Das abgekühlte Reaktionsgemisch wurde auf wässrige Natriumhydrogencarbonat-Lösung gegossen und mit Methylenchlorid versetzt. Die wässrige Phase wurde abgetrennt und noch zweimal mit Methylenchlorid extrahiert. Die vereinigten organischen Phasen wurden zweimal mit Wasser gewaschen, über Magnesiumsulfat getrocknet, filtriert und eingedampft. Umkristallisation des erhaltenen Rohproduktes (5,84 g) aus einem Gemisch von 200 ml Hexan und 25 ml Aethylacetat ergab 4,97 g 4'-[(2S,4R,5S)-5-Octyl-4-methyl-1,3-dioxan-2-yl] -4-cyanobiphenyl mit Smp. (C-$S_A$) 114,5 ° C, Klp. ($S_A$-I) 128,4 ° C.

Das als Ausgangsmaterial verwendete (2S,3R)-2-Octyl-1,3-butandiol wurde wie folgt hergestellt:

a) Ein Gemisch von 83,5 g Diisopropylamin und 400 ml Tetrahydrofuran wurde bei -20 ° C innert 30 Minuten tropfenweise mit 500 ml einer 1,6M Lösung von Butyllithium (0,800 Mol) in Hexan versetzt. Das Gemisch wurde noch 30 Minuten bei -20 ° C bis -5 ° C weitergerührt. Anschliessend wurde das Gemisch bei -50 ° C innert 30 Minuten tropfenweise mit einer Lösung von 50 g (0,378 Mol) (3R)-3-Hydroxybuttersäure-äthylester in 250 ml Tetrahydrofuran versetzt und noch 30 Minuten bei -30 ° C weitergerührt. Danach wurde das Reaktionsgemisch bei -50 ° C innert 30 Minuten tropfenweise mit einer Lösung von 108 g 1-Bromoctan (0,560 Mol) in 96,5 g Hexamethylphosphorsäuretriamid versetzt. Das Reaktionsgemisch wurde auf Raumtemperatur erwärmen gelassen und noch 3 Stunden bei 25 ° C gerührt. Anschliessend wurde die braune Reaktionslösung auf 1,3 l Wasser gegossen und dreimal mit Aethylacetat extrahiert. Die organischen Phasen wurden mit gesättigter Kochsalzlösung gewaschen, über Natriumsulfat getrocknet, filtriert und eingeengt. Hochvakuumdestillation des erhaltenen braunen Oeles (128 g) ergab 54 g (2S,3R)-3-Hydroxy-2-octylbuttersäure-äthylester mit Sdp. 99 ° C/0,03 Torr und $[\alpha]_D$ = +6,2 ° (c = 1% in Chloroform).

b) Eine Suspension von 33,5 g (0,883 Mol) Lithiumaluminiumhydrid in 980 ml Diäthyläther wurde bei Raumtemperatur innert 30 Minuten tropfenweise mit einer Lösung von 54 g (2S,3R)-3-Hydroxy-2-octylbuttersäure-äthylester in 250 ml Diäthyläther versetzt. Anschliessend wurde das Reaktionsgemisch 2,5 Stunden zum Rückfluss erhitzt und dann auf 0 ° C gekühlt. Danach wurde das Reaktionsgemisch tropfenweise mit 100 ml Aceton und dann mit gesättigter Natriumcarbonat-Lösung versetzt, bis sich ein Niederschlag bildete. Die Aetherphase wurde abgegossen und filtriert. Der Rückstand im Reaktionskolben wurde noch zweimal mit je 1 l Diäthyläther verrührt und dann jeweils die Aetherphase abgegossen und filtriert. Die Aetherphasen wurden vereinigt und zur Trockene eingeengt. Hierbei wurden 45 g Rohprodukt als gelbes Oel erhalten. Hochvakuumdestillation bis zu einer Oelbadtemperatur von 160 ° C ergab als Rückstand 40 g (2S,3R)-2-Octyl-1,3-butandiol mit $[\alpha]_D$ = +18,7 ° (c = 1% in Chloroform).

In analoger Weise können folgende Verbindungen hergestellt werden:

4'-[(2S,4R,5S)-5-Decyl-4-methyl-1,3-dioxan-2-yl]-4-cyanobiphenyl, Smp. (C-$S_A$) 115,2 ° C, Klp. ($S_A$-I) 130,6 ° C;

4-[trans-4-[(2S,4R,5S)-5-Octyl-4-methyl-1,3-dioxan-2-yl]-cyclohexyl]benzonitril, Smp. (C-N*) 65,3 ° C, Klp. (N*-I) 91,2 ° C;

4-[trans-4-[(2S,4R,5S)-5-Octyl-4-methyl-1,3-dioxan-2-yl]-cyclohexyl]-1-fluorbenzol, Smp. 63,5 ° C;

4-[trans-4-[(2S,4R,5S)-5-Octyl-4-methyl-1,3-dioxan-2-yl]-cyclohexyl]-1,2-difluorbenzol, Smp. 32,9 ° C;

4-[trans-4-[(2S,4R,5S)-5-Octyl-4-methyl-1,3-dioxan-2-yl]-cyclohexyl]-1-chlorbenzol, Smp. 66,7 ° C;

4-[trans-4-[(2S,4R,5S)-5-Octyl-4-methyl-1,3-dioxan-2-yl]-cyclohexyl]-1-chlor-2-fluorbenzol, Smp. 40 ° C;

4-[trans-4-[(2S,4R,5S)-5-(2-propenyl)-4-methyl-1,3-dioxan2-yl]-cyclohexyl]benzonitril, Smp. 87,2 ° C;

5-[(2S,4R,5S)-5-Octyl-4-methyl-1,3-dioxan-2-yl]-2-(4-decyloxyphenyl)pyrimidin, Smp. (C-N*) 67,5 ° C, Klp. (N*-I) 106,7 ° C;

5-[(2S,4R,5S)-5-Decyl-4-methyl-1,3-dioxan-2-yl]-2-(3-fluor-4-hexyloxyphenyl)pyridin, Smp. (C-N*) 72,2 ° C, Klp. (N*-I) 87,7 ° .

Beispiel 2

In einem Sulfierkolben mit Rührer, Kühler und Wasserabscheider wurden unter Stickstoffbegasung 8,8 g Terephthalaldehyd mit einer Lösung von 25 g (2S,3R)-2-Octyl-1,3-butandiol in 500 ml Toluol versetzt. Anschliessend wurde das Gemisch mit 1 ml 1N Schwefelsäure versetzt und 18 Stunden zum Rückfluss erhitzt. Danach wurde das Reaktionsgemisch auf Raumtemperatur abgekühlt, mit 1,5 ml Trimethylamin versetzt und in 500 ml Diäthyläther und 500 ml gesättigter Natriumhydrogencarbonat-Lösung verteilt. Die wässrige Phase wurde abgetrennt und noch zweimal mit je 300 ml Diäthyläther extrahiert. Die organischen Phasen wurden zweimal mit je 400 ml gesättigter Natriumhydrogencarbonat-Lösung und einmal mit 400 ml Wasser gewaschen, über Natriumsulfat getrocknet, filtriert und eingeengt. Chromatographische Reinigung des erhaltenen Rohproduktes (31 g) an Kieselgel mit Petroläther/Aethylacetat (Vol. 95:5) und zweimalige Umkristallisation aus Hexan ergab 19,1 g 1,4-Bis-[(2S,4R,5S)-5-octyl-4-methyl-1,3-dioxan-2-yl]benzol als weisse Kristalle mit Smp. (C-I) 80,4 °C und $[\alpha]_D$ = +32,0 ° (c = 1% in Chloroform).

Beispiel 3

Ein Gemisch von 4,93 g 4'-[(25,4R,5S)-5-Octyl-4-methyl-1,3-dioxan-2-yl] -4-biphenylcarbonsäure, 2,28 ml (R)-2-Octanol, 3,72 g N,N'-Dicyclohexylcarbodiimid, 205 mg 4-(Dimethylamino)pyridin und 450 ml Methylenchlorid wurde 4 Stunden unter Stickstoffbegasung gerührt. Anschliessend wurde das Reaktionsgemisch filtriert und das Filtrat eingedampft. Das erhaltene Rohprodukt (11,28 g) wurde in 150 ml Methylenchlorid suspendiert. Die Suspension wurde filtriert und das Filtrat eingedampft. Kristallisation der erhaltenen gelben Flüssigkeit (7,62 g) bei -20 °C in Hexan, Abtrennung der kristallinen Verunreinigungen durch Filtration, Eindampfen der Mutterlauge und Filtration über basischem Aluminiumoxid mit Methylenchlorid ergab 4,40 g 4'-[(2S,4R, 5S)-5-Octyl-4-methyl-1,3-dioxan-2-yl] -4-biphenylcarbonsäure-(R)-2-octylester mit Klp. -25 °C und $[\alpha]_D$ = -13,1 ° (c = 0,7% in Chloroform).

Die als Ausgangsmaterial hergestellte 4'-[(2S,4R,5S)-5-Octyl-4-methyl-1,3-dioxan-2-yl] -4-biphenylcarbonsäure wurde wie folgt hergestellt:

4,97 g 4'-[(2S,4R,5S)-5-Octyl-4-methyl-1,3-dioxan-2-yl) -4-cyanobiphenyl wurde unter Stickstoffbegasung mit einer Lösung von 7,1 g Kaliumhydroxid in 70 ml Diäthylenglykol versetzt. Das Gemisch wurde 2,5 Stunden auf 180 °C erhitzt. Danach wurde das Reaktionsgemisch auf 70 °C abgekühlt, mit 150 ml Wasser verdünnt und mit 1N Salzsäure auf pH 9 eingestellt. Die Lösung wurde mit Eis gekühlt, mit 250 ml Methylenchlorid versetzt und mit 1N Salzsäure auf pH 3-3,5 eingestellt. Die wässrige Phase wurde abgetrennt und noch dreimal mit Methylenchlorid extrahiert. Die vereinigten organischen Phasen wurden dreimal mit Wasser gewaschen, über Magnesiumsulfat getrocknet, filtriert und eingedampft. Dies ergab 4,93 g 4'[(2S,4R,5S)-5-Octyl-4-methyl-1,3-dioxan-2-yl] -4-biphenylcarbonsäure als gelblichen, festen Rückstand.

In analoger Weise können folgende Verbindungen hergestellt werden:

4-[(2S,4R,5S)-5-(2-Propenyl)-4-methyl-1,3-dioxan-2-yl]-benzoesäure-(R)-2-octylester, isotropes Oel;

4-[(2S,4R,5S)-5-Octyl-4-methyl-1,3-dioxan-2-yl]benzoesäure-(R)-2-octylester, isotropes Oel;

4-[(2S,4R,5S)-5-Decyl-4-methyl-1,3-dioxan-2-yl]benzosäure-(R)-2-hexylester, isotropes Oel;

4-[(2S,4R,5S)-5-Decyl-4-methyl-1,3-dioxan-2-yl]benzoesäure-(R)-2-octylester, isotropes Oel;

4'-[(2S,4R,5S)-5-Decyl-4-methyl-1,3-dioxan-2-yl]-4-biphenylcarbonsaure-(R)-2-octylester, Smp. (C-I) 21,6 °C;

4'-[(2S,4R,5S)-5-Octyl-4-methyl-1,3-dioxan-2-yl]-4-biphenylcarbonsäure-(R)-1-(isobutyloxycarbonyl)-äthylester, Smp. (C-I) 57,5 °C;

4-[trans-4-[(2S,4R,5S)-5-Octyl-4-methyl-1,3-dioxan-2-yl]cyclohexyl]benzoesäure-(R)-2-octylester, Smp. (C-I) 24,8 °C.

Beispiel 4

Ein Gemisch von 380 mg 4-[(2S,4R,5S)-5-Octyl-4-methyl-1,3-dioxan-2-yl]phenol, 185 mg (S)-2-Fluorcapronsäure, 333 mg N,N'-Dicyclohexylcarbodiimid, 20 mg 4-(Dimethylamino)pyridin und 40 ml Methylenchlorid wurde 16,5 Stunden unter Stickstoff bei Raumtemperatur gerührt. Anschliessend wurde die Suspension filtriert und das Filtrat am Rotationsverdampfer eingedampft. Das Rohprodukt (1,0 g) wurde in 50 ml Methylenchlorid suspendiert, die Suspension filtriert und das Filtrat eingedampft. Umkristallisation des weissen, festen Rückstandes (0,5 g) aus Hexan ergab 0,4 g 1-[(2S,4R, 5S)-5-Octyl-4-methyl-1,3-dioxan-2-yl] -4-[(S)-2-fluorhexanoyloxy]benzol als weisse Kristalle mit Smp. (C-I) 58,8 °C und $[\alpha]_D$ = +16,7 ° (c = 1% in Chloroform).

Das als Ausgangsmaterial verwendete 4-[(2S,4R,5S)-5-Octyl-4-methyl-1,3-dioxan-2-yl]phenol wurde wie folgt hergestellt:

In einem Rundkolben mit Magnetrührer, Wasserabscheider und Kühler wurde unter Stickstoff ein Gemisch von 320 mg (2S,3R)-2-Octyl-1,3-butandiol, 193 mg p-Hydroxybenzaldehyd, 0,1 ml 1N Schwefelsäure und 20 ml Toluol vorgelegt. Das Gemisch wurde unter Wasserabscheidung 3 Stunden zum Rückfluss erhitzt. Das abgekühlte Reaktionsgemisch wurde auf wässrige Natriumhydrogencarbonat-Lösung gegossen und mit Diäthyläther versetzt. Die wässrige Phase wurde abgetrennt und noch dreimal mit Diäthyläther extrahiert. Die vereinigten organischen Phasen wurden dreimal mit Wasser gewaschen, über Magnesiumsulfat getrocknet, filtriert und eingedampft. Umkristallisation des gelblichen, festen Rückstandes (480 mg) aus Hexan/Aethylacetat (Vol. 25:1) ergab 410 mg 4-[(2S,4R,5S)-5-Octyl-4-methyl-1,3-dioxan-2-yl]phenol als gelbliche Kristalle mit $[\alpha]_D$ = +27° (c = 1% in Chloroform).

In analoger Weise können folgende Verbindungen hergestellt werden:

4-Octyloxybenzoesäure-4-[(2S,4R,5S)-5-octyl-4-methyl-1,3-dioxan-2-yl]phenylester, Smp. (C-S) 52°C, S-N* 57,4°C, Klp. (N*-I) 101°C;

4-Hexyloxybenzoesäure-4-[(2S,4R,5S)-5-decyl-4-methyl-1,3-dioxan-2-yl]phenylester, Smp. (C-N*) 77°C, Klp. (N*-I) 101°C;

4-Hexyloxybenzoesäure-4-[(2S,4R,5S)-5-decyl-4-methyl-1,3-dioxan-2-yl]-3-chlorphenylester, Smp. (C-I) 47,5°C.

## Beispiel 5

Eine Lösung von 1,7 g 4'-[(2S,4R,5S)-5-Octyl-4-methyl-1,3-dioxan-2-yl] -4-cyanobiphenyl in 12 ml Toluol wurde bei 0°C unter Stickstoffbegasung innert 10 Minuten mit 4,6 ml Diisobutylaluminiumhydrid versetzt. Das Reaktionsgemisch wurde noch 3 Stunden ohne Kühlung weitergerührt und dann vorsichtig mit Eis/Wasser versetzt. Das Gemisch wurde in Diäthyläther/Wasser verteilt, mit 1N Schwefelsäure auf pH 6 eingestellt und die wässrige Phase abgetrennt und noch dreimal mit Diäthyläther extrahiert. Die organischen Phasen wurden zweimal mit Wasser gewaschen, über Magnesiumsulfat getrocknet, filtriert und eingedampft. Der erhaltene, rohe 4'-[(2S,4R,5S)-5-Octyl-4-methyl-1,3-dioxan-2-yl] -4-biphenylcarboxaldehyd (1,55 g) wurde in analoger Weise zu Beispiel 1 zu 4,4'-Bis-[(2S,4R,5S)-5-octyl-4-methyl-1,3-dioxan-2-yl]-biphenyl umgesetzt; Smp. (C-N*) 95,3°C, Klp. (N*-I) 120,5°C.

## Beispiel 6

Zur Messung der induzierten Ganghöhe (Pitch) und deren Temperaturabhängigkeit in Flüssigkristallmaterialien wurde die folgende FlüssigkristallGrundmischung BM-1 verwendet:

5,36 Gew.% 4'-Aethyl-4-cyanobiphenyl,
3,18 Gew.% 4'-Propyl-4-cyanobiphenyl,
6,08 Gew.% 4'-Butyl-4-cyanobiphenyl,
6,53 Gew.% 4-(trans-4-Propylcyclohexyl)benzonitril,
14,67 Gew.% 4-(trans-4-Pentylcyclohexyl)benzonitril,
5,21 Gew.% 4-Aethyl-1-(trans-4-propylcyclohexyl)benzol,
16,54 Gew.% 4-Aethoxy-1-[2-(trans-4-propylcyclohexyl)äthyl]benzol,
5,60 Gew.% 4"-Pentyl-4-cyano-p-terphenyl,
5,71 Gew.% 4'-(trans-4-Pentylcyclohexyl)-4-cyanobiphenyl,
15,95 Gew.% 1-[2-(trans-4-Butylcyclohexyl)äthyl]-4-(trans-4-pentylcyclohexyl)benzol,
4,74 Gew.% 4-[2-(trans-4-Butylcyclohexyl)äthyl]-4'-(trans-4-pentylcyclohexyl)biphenyl,
7,59 Gew.% 4-[2-(trans-4-Butylcyclohexyl)äthyl]-4'-(trans-4-pentylcyclohexyl)-1,1'-äthylendibenzol,
2,84 Gew.% trans-4-[2-(trans-4-Propylcyclohexyl)äthyl]-cyclohexancarbonsäure-4-cyanophenylester;
Smp. <-30°C, Klp. (N-I) 90°C; $\Delta\epsilon$ = 8,5, $\Delta n$ = 0,139 und $\eta$ = 22 mPa•s gemessen bei 22°C.

Das Verdrillungsvermögen des optisch aktiven Dotierstoffes und dessen Temperaturabhängigkeit wird charakterisiert durch die Parameter A, B und C entsprechend der Reihenentwicklung.

$$\frac{1}{pc} = A + BT_1 + CT_1^2$$

worin und p, c und $T_1$ die folgenden Bedeutungen haben:

$T_1$ = T-22°C

T = Temperatur in °C

p = Ganghöhe in µm (ein positiver Wert bedeutet rechtsdrehende Helixstruktur und ein negativer Wert bedeutet linksdrehende Helixstruktur)

c = Konzentration des optisch aktiven Dotierstoffes in Gew.-%.

Grundmischung BM-1 (99 Gew.-%) wurde jeweils mit 1 Gew.-% eines der folgenden Dotierstoffe der Formel I gemischt:

D-1 = 4'-[(2S,4R,5S)-5-Octyl-4-methyl-1,3-dioxan-2-yl]-4-biphenylcarbonsäure-(R)-2-octylester,

D-2 = 4-[(2S,4R,5S)-5-Decyl-4-methyl-1,3-dioxan-2-yl]-benzoesäure-(R)-2-octylester,

D-3 = 4-[(2S,4R,5S)-5-Octyl-4-methyl-1,3-dioxan-2-yl]-benzoesäure-(R)-2-octylester,

D-4 = 4'-[(2S,4R,5S)-5-Decyl-4-methyl-1,3-dioxan-2-yl]-4-cyanobiphenyl,

D-5 = 4'-[(2S,4R,5S)-5-Decyl-4-methyl-1,3-dioxan-2-yl]-4-biphenylcarbonsäure-(R)-2-octylester,

D-6 = 4,4'-Bis-[(2S,4R,5S)-5-octyl-4-methyl-1,3-dioxan-2-yl]biphenyl,

D-7 = 1,4-Bis-[(2S,4R,5S)-5-octyl-4-methyl-1,3-dioxan-2-yl]benzol,

D-8 = 4-[trans-4-[(2S,4R,5S)-5-Octyl-4-methyl-1,3-dioxan-2-yl]cyclohexyl]benzonitril.

Die Helixganghöhe p bei 22°C, die Aenderung $\Delta T_c$ des Klärpunktes und die Werte von A, B und C der erhaltenen cholesterischen Gemische sind in Tabelle 1 angegeben.

Tabelle 1

| Dotierstoff | p [µm] | $\Delta T_c$ [°C] | A [$10^{-2} \cdot µm^{-1} \cdot$ Gew.-%$^{-1}$] | B [$10^{-4} \cdot µm^{-1} \cdot$ Gew.-%$^{-1} \cdot °C^{-1}$] | C [$10^{-6} \cdot µm^{-1} \cdot$ Gew.-%$^{-1} \cdot °C^{-2}$] |
|---|---|---|---|---|---|
| D-1 | 6,79 | -0,9 | 14,72 | 1,757 | -1,138 |
| D-2 | 6,5 | -1,1 | 15,42 | -2,946 | -4,192 |
| D-3 | 6,30 | -4,2 | 15,864 | 0,703 | 0,789 |
| D-4 | 14,9 | -1,5 | 6,699 | 0,832 | 0,444 |
| D-5 | 7,5 | -2,5 | 13,52 | 1,315 | -1,480 |
| D-6 | 12,2 | -0,3 | 8,476 | 2,806 | 1,028 |
| D-7 | 7,4 | -1,8 | 16,26 | 2,729 | -2,461 |
| D-8 | 15,8 | -1,4 | 6,308 | -0,328 | 0,690 |

Beispiel 7

Die folgenden cholesterischen Mischungen CM-1 bis CM-6 illustrieren die Verwendung mehrerer chiraler Dotierstoffe zur Erzielung einer kurzen Helixganghöhe mit geringer Temperaturabhängigkeit. Hierzu

wurden mehrere nematische Grundmischungen mit einer Kombination von 3 bzw. 4 chiralen Dotierstoffen gemischt. Die erhaltenen cholesterischen Mischungen eignen sich insbesondere für cholesterische Filter und zeichnen sich durch eine geringe Temperaturabhängigkeit der Wellenlänge $\lambda_{max}$ des selektiv reflektierten, zirkular polarisierten Lichts aus. Die bei verschiedenen Temperaturen T gemessenen Werte von $\lambda_{max}$ sind in Tabelle 2 zusammengestellt. Die in den Mischungsbeispielen verwendeten Bezeichnungen der Komponenten besitzen folgende Bedeutungen:

| | |
|---|---|
| 2PP = | 4'-Aethyl-3-cyanobiphenyl, |
| 3PP = | 4'-Propyl-4-cyanobiphenyl, |
| 4PP = | 4'-Butyl-4-cyanobiphenyl, |
| 3CP = | 4-(trans-4-Propylcyclohexyl)benzonitril, |
| 5CP = | 4-(trans-4-Pentylcyclohexyl)benzonitril, |
| 3CP2 = | 4-Aethyl-1-(trans-4-propylcyclohexyl)benzol, |
| 3CAPO2 = | 4-Aethoxy-1-[2-(trans-4-propylcyclohexyl)äthyl]benzol, |
| 5CAPO2 = | 4-Aethoxy-1-[2-(trans-4-pentylcyclohexyl)äthyl]benzol, |
| 5PPP = | 4"-Pentyl-4-cyano-p-terphenyl, |
| 5CPP = | 4'-(trans-4-Pentylcyclohexyl)-4-cyanobiphenyl, |
| 5CPAC4 = | 1-[2-(trans-4-Butylcyclohexyl)äthyl]-4-(trans-4-pentylcyclohexyl)benzol, |
| 5CPPAC4 = | 4-[2-(trans-4-Butylcyclohexyl)äthyl]-4'-(trans-4-pentylcyclohexyl)biphenyl, |
| 5CPAPAC4 = | 4-[2-(trans-4-Butylcyclohexyl)äthyl]-4'-(trans-4-pentylcyclohexyl)-1,1'-äthylendibenzol, |
| 3CACEP = | trans-4-[2-(trans-4-Propylcyclohexyl)äthyl]cyclohexancarbonsäure-4-cyanophenylester, |
| 1d(3)CCO1 = | trans-4-Methoxy-1-[trans-4-(3E-pentenyl)cyclohexyl]cyclohexan, |
| 1d(1)CC101 = | trans-4-Methoxymethyl-1-[trans-4-(1E-propenyl)cyclohexyl]cyclohexan, |
| 0d(3)CCO2 = | trans-4-Aethoxy-1-[trans-4-(3-butenyl)cyclohexyl]cyclohexan, |
| 0d(4)CCO2 = | trans-4-Aethoxy-1-[trans-4-(4-pentenyl)cyclohexyl]cyclohexan, |
| 1d(3)CPO2 = | 4-Aethoxy-1-[trans-4-(3E-pentenyl)cyclohexyl]benzol, |
| 3CPOd(3)1 = | 4-(2E-Butenyloxy)-1-(trans-4-propylcyclohexyl)benzol, |
| 5CPOd(3)1 = | 4-(2E-Butenyloxy)-1-(trans-4-pentylcyclohexyl)benzol, |
| 1d(3)CPP2 = | 4'-Aethyl-4-[trans-4-(3E-pentenyl)cyclohexyl]biphenyl, |
| 1d(3)CPP3 = | 4'-Propyl-4-[trans-4-(3E-pentenyl)cyclohexyl]biphenyl, |
| 3CEPCd(3)1 = | trans-4-Propylcyclohexancarbonsäure-4-[trans-4-(3E-pentenyl)cyclohexyl]phenylester, |
| 0d(3)CP = | 4-[trans-4-(3-Butenyl)cyclohexyl]benzonitril, |
| 1d(1)CP = | 4-[trans-4-(1E-Propenyl)cyclohexyl]benzonitril, |
| 4PP(1)P5 = | 5-(4-Butylphenyl)-2-(4-pentylphenyl)pyrimidin, |
| 2CP(1)P5 = | 5-(trans-4-Aethylcyclohexyl)-2-(4-pentylphenyl)pyrimidin, |
| 3PPt1 = | 4-(1-Propinyl)-4'-propylbiphenyl, |
| D-7 = | 1,4-Bis-[(2S,4R,5S)-5-octyl-4-methyl-1,3-dioxan-2-yl]benzol, |
| D-2 = | 4-[(2S,4R,5S)-5-Decyl-4-methyl-1,3-dioxan-2-yl]benzoesäure-(R)-2-octylester, |
| D-10 = | (4S,5S)-2-[trans-4-(4-Cyanophenyl)cyclohexyl]-1,3-dioxolan-4,5-dicarbonsäurediäthylester, |
| D-11 = | (S)-4'-(2-Methylbutyl)-4-cyanobiphenyl. |

Mischung CM-1

4,350 Gew.-% 2PP
2,580 Gew.-% 3PP
4,930 Gew.-% 4PP
5,300 Gew.-% 3CP
11,900 Gew.-% 5CP
4,230 Gew.-% 3CP2
13,420 Gew.-% 3CAPO2
4,540 Gew.-% 5PPP
4,630 Gew.-% 5CPP
12,940 Gew.-% 5CPAC4
3,850 Gew.-% 5CPPAC4
6,160 Gew.-% 5CPAPAC4
2,310 Gew.-% 3CACEP
7,700 Gew.-% D-7

7,310 Gew.-% D-10
3,850 Gew.-% D-2

Mischung CM-2

4,220 Gew.-% 2PP
2,500 Gew.-% 3PP
4,780 Gew.-% 4PP
5,140 Gew.-% 3CP
11,540 Gew.-% 5CP
4,100 Gew.-% 3CP2
13,020 Gew.-% 3CAPO2
4,410 Gew.-% 5PPP
4,490 Gew.-% 5CPP
12,550 Gew.-% 5CPAC4
3,730 Gew.-% 5CPPAC4
5,970 Gew.-% 5CPAPAC4
2,240 Gew.-% 3CACEP
8,700 Gew.-% D-7
8,260 Gew.-% D-10
4,350 Gew.-% D-2

Mischung CM-3

3,190 Gew.-% 1d(3)CCO1
7,960 Gew.-% 1d(1)CC101
5,570 Gew.-% 0d(3)CCO2
3,180 Gew.-% 0d(4)CCO2
2,390 Gew.-% 1d(3)CPO2
3,980 Gew.-% 3CPOd(3)1
4,780 Gew.-% 5CPOd(3)1
4,780 Gew.-% 3CAPO2
4,780 Gew.-% 5CAPO2
3,980 Gew.-% 1d(3)CPP2
5,570 Gew.-% 1d(3)CPP3
6,370 Gew.-% 5CPAC4
3,980 Gew.-% 3CEPCd(3)1
3,980 Gew.-% 0d(3)CP
3,180 Gew.-% 1d(1)CP
2,390 Gew.-% 4PP(1)P5
2,390 Gew.-% 2CP(1)P5
2,390 Gew.-% 5CPPAC4
4,780 Gew.-% 3PPt1
6,710 Gew.-% D-7
6,320 Gew.-% D-10
4,190 Gew.-% D-2
3,160 Gew.-% D-11

Mischung CM-4

3,070 Gew.-% 1d(3)CO1
7,680 Gew.-% 1d(1)CC101
5,380 Gew.-% 0d(3)CCO2
3,070 Gew.-% 0d(4)CCO2
2,300 Gew.-% 1d(3)CP02
3,840 Gew.-% 3CPOd(3)1
4,610 Gew.-% 5CPOd(3)1
4,610 Gew.-% 3CAPO2

4,610 Gew.-% 5CAPO2
3,840 Gew.-% 1d(3)CPP2
5,370 Gew.-% 1d(3)CPP3
6,140 Gew.-% 5CPAC4
3,840 Gew.-% 3CEPCd(3)1
3,840 Gew.-% 0d(3)CP
3,070 Gew.-% 1d(1)CP
2,300 Gew.-% 4PP(1)P5
2,300 Gew.-% 2CP(1)P5
2,300 Gew.-% 5CPPAC4
4,610 Gew.-% 3PPt1
7,650 Gew.-% D-7
7,200 Gew.-% D-10
4,770 Gew.-% D-2
3,600 Gew.-% D-11

Mischung CM-5

2,970 Gew.-% 1d(3)CCO1
7,420 Gew.-% 1d(1)CC101
5,190 Gew.-% 0d(3)CCO2
2,970 Gew.-% 0d(4)CCO2
2,230 Gew.-% 1d(3)CP02
3,710 Gew.-% 3CPOd(3)1
4,450 Gew.-% 5CPOd(3)1
4,450 Gew.-% 3CAPO2
4,450 Gew.-% 5CAPO2
3,710 Gew.-% 1d(3)CPP2
5,190 Gew.-% 1d(3)CPP3
5,930 Gew.-% 5CPAC4
3,710 Gew.-% 3CEPCd(3)1
3,710 Gew.-% 0d(3)CP
2,970 Gew.-% 1d(1)CP
2,230 Gew.-% 4PP(1)P5
2,230 Gew.-% 2CP(1)P5
2,230 Gew.-% 5CPPAC4
4,450 Gew.-% 3PPt1
8,500 Gew.-% D-7
8,000 Gew.-% D-10
5,300 Gew.-% D-2
4,000 Gew.-% D-11

Mischung CM-6

4,050 Gew.-% 2PP
2,400 Gew.-% 3PP
4,590 Gew.-% 4PP
4,930 Gew.-% 3CP
11,080 Gew.-% 5CP
3,930 Gew.-% 3CP2
12,490 Gew.-% 3CAPO2
4,230 Gew.-% 5PPP
4,310 Gew.-% 5CPP
12,040 Gew.-% 5CPAC4
3,580 Gew.-% 5CPPAC4
5,730 Gew.-% 5CPAPAC4
2,140 Gew.-% 3CACEP
10,000 Gew.-% D-7

9,500 Gew.-% D-10
5,000 Gew.-% D-2

Tabelle 2

| T [°C] | $\lambda_{max}$ [nm] | | | | | |
|---|---|---|---|---|---|---|
| | CM-1 | CM-2 | CM-3 | CM-4 | CM-5 | CM-6 |
| 0 | 610 | 527 | 616 | 540 | 467 | 456 |
| 10 | 610 | 527 | 614 | 540 | 467 | 460 |
| 20 | 610 | 534 | 613 | 540 | | |
| 22 | | | | | 471 | 464 |
| 30 | 618 | 535 | 612 | 541 | 471 | 470 |
| 40 | 621 | 540 | 611 | 540 | 476 | 473 |
| 50 | 636 | 548 | 624 | 544 | 478 | 477 |

Beispiel 8

Die in Tabelle 3 aufgeführten, chiral getilteten smektischen Mischungen SM-1 bis SM-3 illustrieren die Verwendung der chiralen Dotierstoffe der Formel I in einem getilteten smektischen Flüssigkristall. 1/(pc) charakterisiert das Verdrillungsvermögen des chiralen Dotierstoffes, wobei p und c die in Beispiel 5 gegebenen Bedeutungen haben. $P_s$ bezeichnet die spontane Polarisation (extrapoliert auf 100 Gew.-%). Die in Tabelle 3 angegebenen Konzentrationen sind in Gew.-%. Die für die Komponenten verwendeten Bezeichnungen besitzen folgende Bedeutungen:

10OPEPAC5 = 4-Decyloxybenzoesäure-4-[2-(trans-4-pentylcyclohexyl)äthyl]phenylester,
11OPEPAC5 = 4-Undecyloxybenzoesäure-4-[2-(trans-4-pentylcyclohexyl)äthyl]phenylester,
12OPEPAC5 = 4-Dodecyloxybenzoesäure-4-[2-(trans-4-pentylcyclohexyl)äthyl]phenylester,
7P(1)PO8 = 2-(4-Octyloxyphenyl)-5-heptylpyrimidin,
9P(1)PO6 = 2-(4-Hexyloxyphenyl)-5-nonylpyrimidin,
9P(1)PO9 = 2-(4-Nonyloxyphenyl)-5-nonylpyrimidin,
10P(1)PO10 = 2-(4-Decyloxyphenyl)-5-decylpyrimidin,
D-12 = 4-Octyloxybenzoesäure-4-[(2S,4R,5S)-5-octyl-4-methyl-1,3-dioxan-2-yl]phenylester,
D-13 = 4-Hexyloxybenzoesäure-4-[(2S,4R,5S)-5-decyl-4-methyl-1,3-dioxan-2-yl]phenylester,
D-14 = 4-Hexyloxybenzoesäure-4-[(2S,4R,5S)-5-decyl-4-methyl-1,3-dioxan-2-yl]-3-chlorphenylester.

Tabelle 3

| | SM-1 | SM-2 | SM-3 |
|---|---|---|---|
| 10OPEPAC5 | 23,4% | 23,2% | 23,4% |
| 11OPEPAC5 | 11,7% | 11,6% | 11,7% |
| 12OPEPAC5 | 11,7% | 11,6% | 11,7% |
| 7P(1)PO8 | 4,3% | 4,3% | 4,3% |
| 9P(1)PO6 | 14,6% | 14,6% | 14,7% |
| 9P(1)PO9 | 14,6% | 14,6% | 14,7% |
| 10P(1)PO10 | 12,7% | 12,6% | 12,8% |
| D-12 | 7,0% | - | - |
| D-13 | - | 7,5% | - |
| D-14 | - | - | 6,5% |
| 1/(pc) [$\mu m^{-1}$•Gew.-$\%^{-1}$] | +12 | +4,5 | +8,3 |
| $P_s$ [$nC/cm^2$] | -67 | -33 | +24 |
| Uebergangs-temperatur[°C] | 59,9($S_C$*-N*) 98,8 (N*-I) | 58($S_C$*-$S_A$) | 51($S_C$*-$S_A$) |

**Patentansprüche**

**1.** Optische aktive Verbindungen der allgemeinen Formel

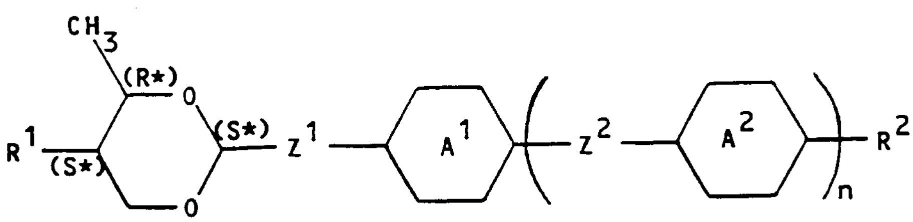

I

worin n für die Zahl 0 oder 1 steht; $Z^1$ eine einfache Kovalenzbindung oder $-CH_2CH_2-$ bezeichnet; $Z^2$ eine einfache Kovalenzbindung, $-CH_2CH_2-$, $-CH_2O-$, $-OCH_2-$, -COO- oder -OOC- bezeichnet; die Ringe $A^1$ und $A^2$ unabhängig voneinander trans-1,4-Cyclohexylen oder gegebenenfalls mit Halogen, Cyano und/oder Methyl substituiertes 1,4-Phenylen, in welchem gegebenenfalls 1 oder 2 CH-Gruppen durch Stickstoff ersetzt sind, darstellen; $R^2$ eine Gruppe $R^4$ oder eine Gruppe der allgemeinen Formel

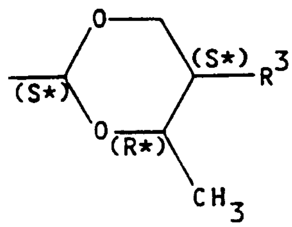

II

bezeichnet; $R^4$ Cyano, Halogen, $-OCY^1F_2$, $-CY^1F_2$ oder eine Alkylgruppe mit 1-18 Kohlenstoffatomen bedeutet, in welcher gegebenenfalls eine >CH-CH< durch >C = C< ersetzt ist und/oder gegebenenfalls eine oder zwei nicht benachbarte Methylengruppen durch -O-, -COO- und/oder -OOC- ersetzt sind und/oder gegebenenfalls eine Methylengruppe durch $-CHY^2-$ ersetzt ist; $Y^1$ Wasserstoff oder Fluor bezeichnet; $Y^2$ Halogen, Cyano oder Methyl bedeutet; $R^1$ und $R^3$ Alkyl mit 1-18 Kohlenstoffatomen bezeichnen, in welchem gegebenenfalls eine >CH-CH< durch >C = C< ersetzt ist und/oder gegebenenfalls eine oder zwei nicht benachbarte Methylengruppen durch -O- ersetzt sind und/oder gegebenenfalls ein oder mehrere Wasserstoffatome durch Fluor ersetzt sind; und (S*) und (R*) die relativen Konfigurationen der chiralen Kohlenstoffatome bezeichnen.

2. Verbindungen nach Anspruch 1, dadurch gekennzeichnet, dass Ring $A^2$ 1,4-Phenylen oder halogensubstituiertes 1,4-Phenylen darstellt, $Z^2$ eine einfache Kovalenzbindung, $-CH_2CH_2-$ oder -OOC- bezeichnet und $Z^1$ eine einfache Kovalenzbindung bezeichnet.

3. Verbindungen nach Anspruch 1 oder 2, dadurch gekennzeichnet, dass Ring $A^1$ Pyridin-2,5-diyl, Pyrimidin-2,5-diyl, trans-1,4-Cyclohexylen oder gegebenenfalls mit Halogen, Cyano und/oder Methyl substituiertes 1,4-Phenylen darstellen.

4. Verbindungen nach einem der Ansprüche 1 bis 3, gekennzeichnet durch die allgemeinen Formeln

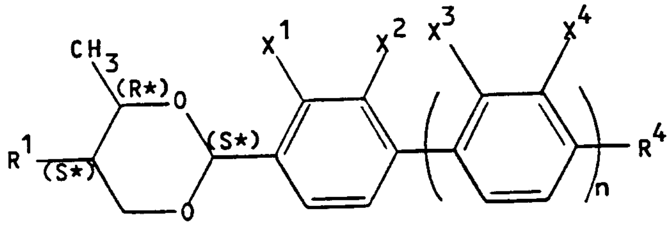

I-1

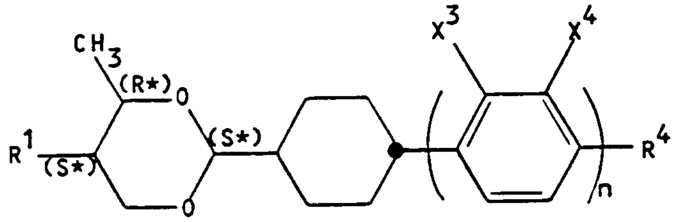

I-2

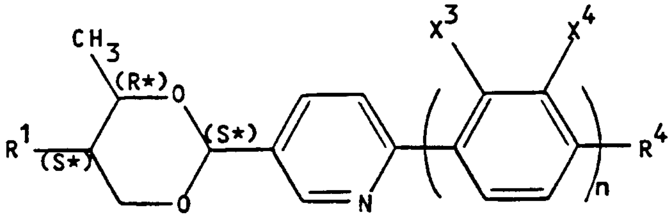

I-3

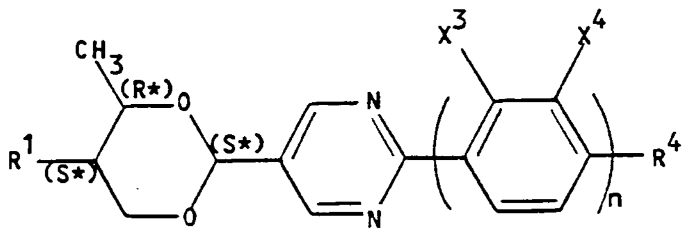

I-4

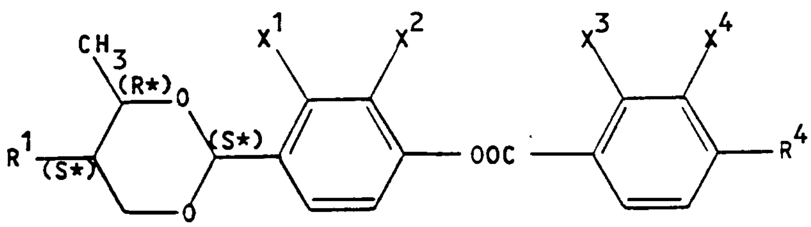

I-5

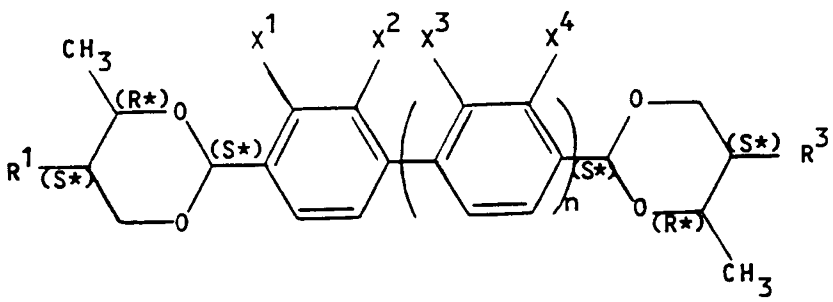

I-6

worin $R^4$ Cyano,Halogen, $-OCY^1F_2$, $-CY^1F_2$ oder eine Alkylgruppe bedeutet, in welcher gegebenenfalls eine >CH-CH< durch >C=C< ersetzt ist und/oder gegebenenfalls eine oder zwei nicht benachbarte Methylengruppen durch -O-, -COO- und/oder -OOC- esetzt sind und/oder gegebenenfalls eine Methylengruppe durch $-CHY^2-$ ersetzt ist; $Y^1$ Wasserstoff oder Fluor bezeichnet; $Y^2$ Halogen, Cyano oder Methyl bedeutet $X^1$, $X^2$, $X^3$ und $X^4$ unabhängig voneinander Wasserstoff oder Halogen bezeichnen; und n, $R^1$, $R^3$, (S*) und (R*) die obigen Bedeutungen haben.

**5.** Verbindungen nach Anspruch 4, dadurch gekennzeichnet, dass $X^1$, $X^2$, $X^3$ und $X^4$ unabhängig voneinander Wasserstoff, Fluor oder Chlor bedeuten.

**6.** Verbindungen nach Anspruch 5, dadurch gekennzeichnet, dass $X^1$ Wasserstoff, Fluor oder Chlor bedeutet, $X^2$ und $X^3$ Wasserstoff bedeuten und $X^4$ Wasserstoff, Fluor oder Chlor bedeutet.

**7.** Verbindungen nach einem der Ansprüche 1 bis 6, dadurch gekennzeichnet, dass $R^1$ und $R^3$ Alkyl bedeuten.

**8.** Verbindungen nach einem der Ansprüche 1 bis 7, dadurch gekennzeichnet, dass $R^4$ Cyano, Fluor, Chlor, Difluormethoxy, Trifluormethoxy, Difluormethyl, Trifluormethyl, Alkyl, Alkoxy, Alkoxycarbonyl, 1-(Alkoxycarbonyl)äthoxycarbonyl, Alkanoyloxy, 2-Fluoralkanoyloxy, Alkenyl oder Alkenyloxy bedeutet.

**9.** Verbindungen nach einem der Ansprüche 1 bis 8, dadurch gekennzeichnet, dass $R^1$, $R^3$ und $R^4$ 1-18, vorzugsweise 1-12 Kohlenstoffatome aufweisen.

**10.** Flüssigkristallines Gemisch mit mindestens zwei Komponenten, dadurch gekennzeichnet, dass mindestens eine Komponente eine optisch aktive Verbindung der in Anspruch 1 definierten Formel I ist.

**11.** Flüssigkristallines Gemisch nach Anspruch 10, dadurch gekennzeichnet, dass es eine oder mehrere optisch aktive Verbindungen der Formel I und ein flüssigkristallines Trägermaterial mit nematischer, cholesterischer oder getilteter smektischer Phase enthält.

12. Flüssigkristallines Gemisch nach Anspruch 10 oder 11, dadurch gekennzeichnet, dass der Anteil an Verbindungen der Formel I 0,1-40 Gew.-% beträgt.

13. Verwendung flüssigkristalliner Gemische gemäss Anspruch 10 für optische oder elektro-optische Zwecke.

**Claims**

1. Optically active compounds of the general formula

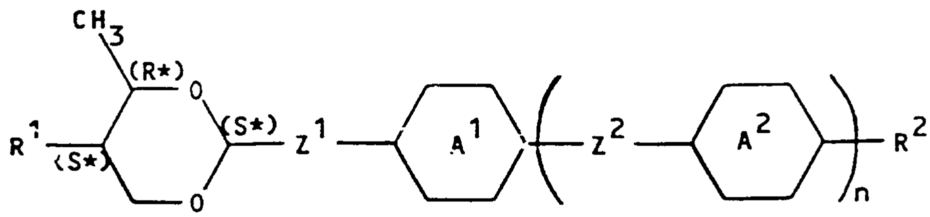

I

wherein n stands for the number 0 or 1; $Z^1$ denotes a single covalent bond or $-CH_2CH_2-$; $Z^2$ denotes a single covalent bond, $-CH_2CH_2-$, $-CH_2O-$, $-OCH_2-$, -COO- or -OOC-; rings $A^1$ and $A^2$ each independently represent trans-1,4-cyclohexylene or optionally halogen-, cyano- and/or methyl-substituted 1,4-phenylene in which optionally 1 CH or 2 CH groups is/are replaced by nitrogen; $R^2$ denotes a group $R^4$ or a group of the general formula

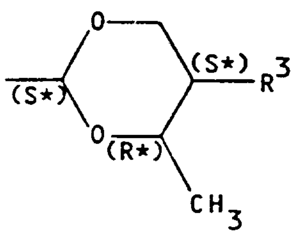

II

$R^4$ signifies cyano, halogen, $-OCY^1F_2$, $-CY^1F_2$ or an alkyl group with 1-18 carbon atoms in which optionally one >CH-CH< is replaced by >C=C< and/or optionally one methylene group or two non-adjacent methylene groups is/are replaced by -O-, -COO- and/or -OOC- and/or optionally one methylene group is replaced by $-CHY^2-$; $Y^1$ denotes hydrogen or fluorine; $Y^2$ signifies halogen, cyano or methyl; $R^1$ and $R^3$ denote alkyl with 1-18 carbon atoms in which optionally one >CH-CH< is replaced by >C=C< and/or optionally one methylene group or two non-adjacent methylene groups is/are replaced by -O- and/or optionally one or more hydrogen atoms is/are replaced by fluorine; and (S*) and (R*) denote the relative configurations of the chiral carbon atoms.

2. Compounds according to claim 1, characterized in that ring $A^2$ represents 1,4-phenylene or halogen-substituted 1,4-phenylene, $Z^2$ denotes a single covalent bond, $-CH_2CH_2-$ or -OOC-and $Z^1$ denotes a single covalent bond.

3. Compounds according to claim 1 or 2, characterized in that ring $A^1$ represents pyridine-2,5-diyl, pyrimidine-2,5-diyl, trans-1,4-cyclohexylene or optionally halogen-, cyano- and/or methyl-substituted 1,4-phenylene.

**4.** Compounds according to any one of claims 1 to 3, characterized by the general formulae

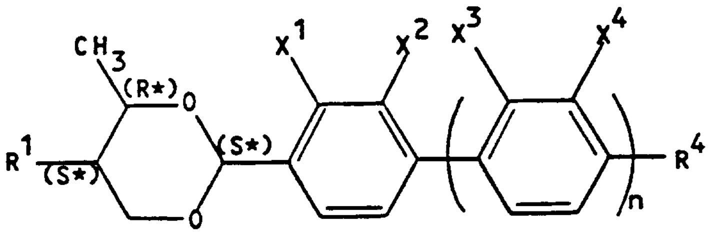

I-1

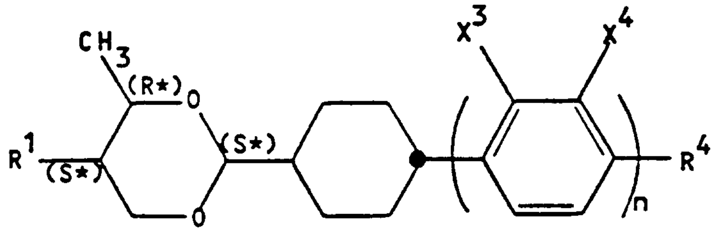

I-2

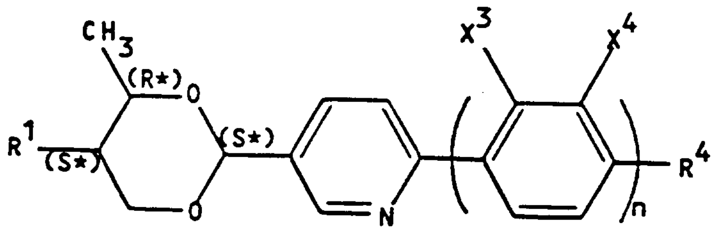

I-3

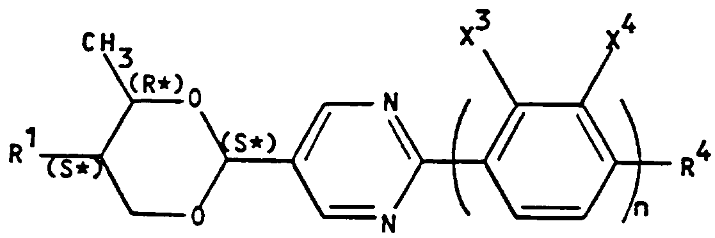

I-4

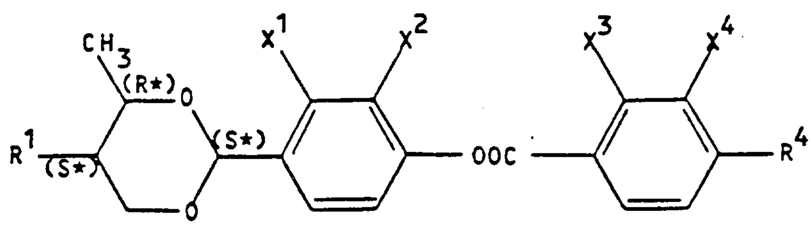

I-5

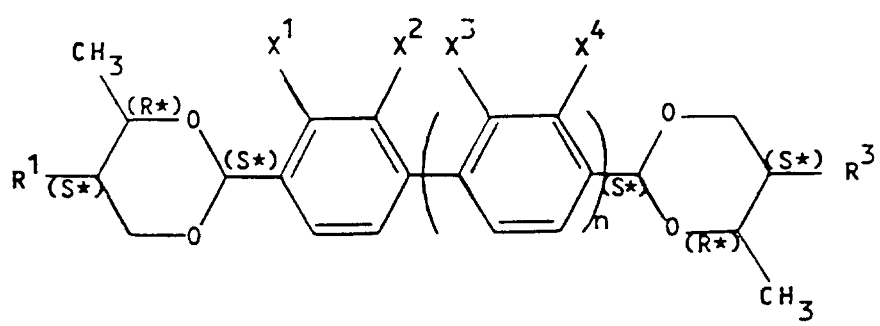

I-6

wherein $R^4$ signifies cyano, halogen, $-OCY^1F_2$, $-CY^1F_2$ or an alkyl group in which optionally one >CH-CH< is replaced by >C=C< and/or optionally one methylene group or two non-adjacent

methylene groups is/are replaced by -O-, -COO-and/or -OOC- and/or optionally one methylene group is replaced by -$CHY^2$-; $Y^1$ denotes hydrogen or fluorine; $Y^2$ signifies halogen, cyano or methyl; $X^1$, $X^2$, $X^3$ and $X^4$ each independently denote hydrogen or halogen; and n, $R^1$, $R^3$, (S*) and (R*) have the above significances.

**5.** Compounds according to claim 4, characterized in that $X^1$, $X^2$, $X^3$ and $X^4$ each independently signify hydrogen, fluorine or chlorine.

**6.** Compounds according to claim 5, characterized in that $X^1$ signifies hydrogen, fluorine or chlorine, $X^2$ and $X^3$ signify hydrogen and $X^4$ signifies hydrogen, fluorine or chlorine.

**7.** Compounds according to any one of claims 1 to 6, characterized in that $R^1$ and $R^3$ signify alkyl.

**8.** Compounds according to any one of claims 1 to 7, characterized in that $R^4$ signifies cyano, fluorine, chlorine, difluoromethoxy, trifluoromethoxy, difluoromethyl, trifluoromethyl, alkyl, alkoxy, alkoxycarbonyl, 1-(alkoxycarbonyl)ethoxycarbonyl, alkanoyloxy, 2-fluoroalkanoyloxy, alkenyl or alkenyloxy.

**9.** Compounds according to any one of claims 1 to 8, characterized in that $R^1$, $R^3$ and $R^4$ have 1-18, preferably 1-12, carbon atoms.

**10.** A liquid crystalline mixture having at least two components, characterized in that at least one component is an optically active compound of formula I defined in claim 1.

**11.** A liquid crystalline mixture according to claim 10, characterized in that it contains one or more optically active compounds of formula I and a liquid crystalline carrier material having a nematic, cholesteric or tilted smectic phase.

**12.** A liquid crystalline mixture according to claim 10 or 11, characterized in that the amount of compounds of formula I amounts to 0.1-40 wt.%.

**13.** The use of liquid crystalline mixtures in accordance with claim 10 for optical or electro-optical purposes.

**Revendications**

**1.** Composés optiquement actifs de formule générale

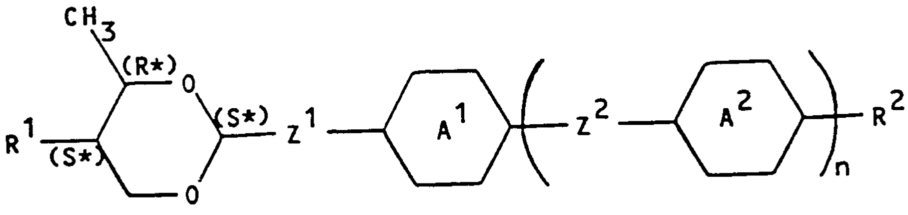

I

dans laquelle n représente le nombre 0 ou 1 ; $Z^1$ représente une liaison de covalence simple ou -$CH_2CH_2$- ; $Z^2$ représente une liaison de covalence simple, -$CH_2CH_2$-,-$CH_2O$-, -$OCH_2$-, -COO- ou -OOC- ; les cycles $A^1$ et $A^2$ représentent indépendamment l'un de l'autre un trans-1,4-cyclohexylène ou un 1,4-phénylène substitué le cas échéant avec un halogène, cyano et/ou méthyle, dans lequel le cas échéant un ou deux groupes CH sont remplacés par l'azote ; $R^2$ représente un groupe $R^4$ ou un groupe de formule générale

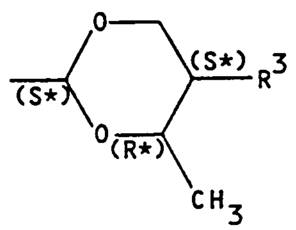

II

$R^4$ représente un cyano, halogène, $-OCY^1F_2$, $-CY^1F_2$ ou un groupe alkyle ayant 1-18 atomes de carbone, dans lequel le cas échéant un groupe >CH-CH< est remplacé par un groupe >C=C< et/ou le cas échéant un ou deux groupes méthylène non-voisins sont remplacés par -O-, -COO-et/ou -OOC- et/ou le cas échéant un groupe méthylène est remplacé par $-CHY^2-$ ; $Y^1$ représente l'hydrogène ou le fluor ; $Y^2$ représente un halogène, un cyano ou un méthyle ; $R^1$ et $R^3$ représentent un alkyle avec 1-18 atomes de carbone, dans lequel le cas échéant un groupe >CH-CH< est remplacé par un groupe >C=C< et/ou le cas échéant un ou deux groupes méthylène non-voisins sont remplacés par -O- et/ou le cas échéant un ou plusieurs atomes d'hydrogène sont remplacés par un fluor; et (S*) et (R*) représentent les configurations relatives des atomes de carbone chiraux.

**2.** Composés selon la revendication 1, caractérisés en ce que le cycle $A^2$ représente un 1,4-phénylène ou un 1,4-phénylène substitué par un halogène; $Z^2$ est une liaison de covalence simple, $-CH_2CH_2-$ ou -OOC- et $Z^1$ représente une liaison covalente simple.

**3.** Composés selon la revendication 1 ou 2 caractérisés en ce que le cycle $A^1$ représente un pyridin-2,5-diyle, pyrimidin-2,5-diyle, trans-1,4-cyclohexylène ou un 1,4-phénylène substitué le cas échéant par un halogène, cyano et/ou méthyle.

**4.** Composés selon l'une des revendications 1 à 3, caractérisés par les formules générales

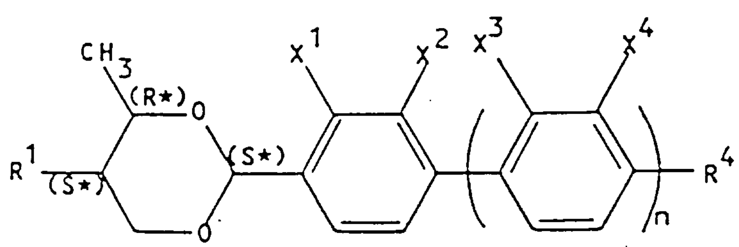

I-1

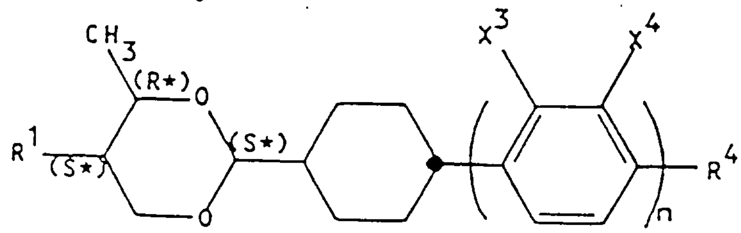

I-2

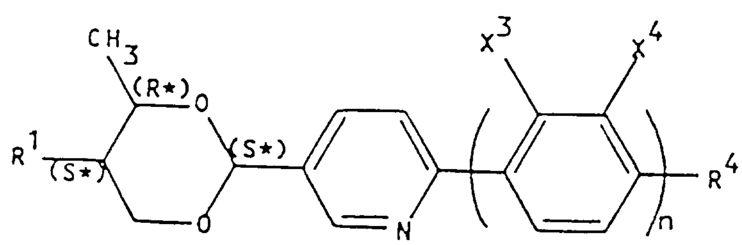

I-3

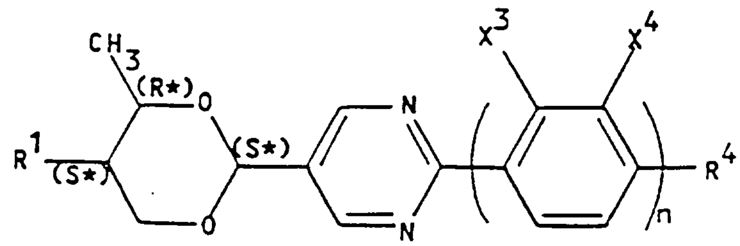

I-4

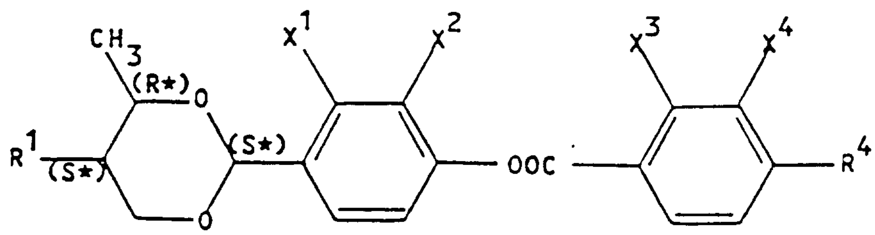

I-5

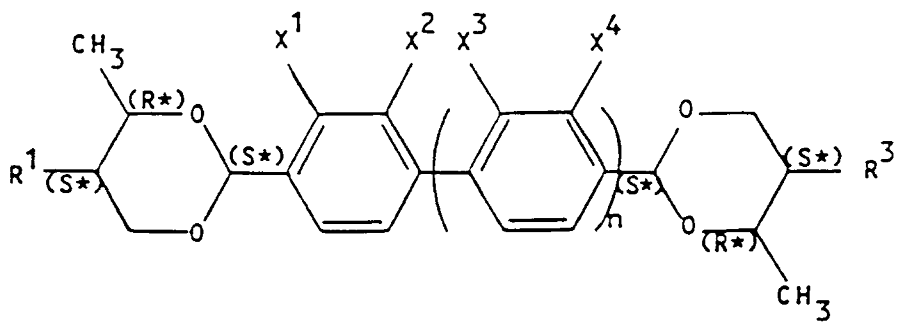

I-6

dans lesquelles $R^4$ représente un cyano, halogène, $OCY^1F_2$, $CY^1F_2$ ou un groupe alkyle, dans lequel le cas échéant un groupe >CH-CH< est remplacé par un groupe >C=C< et/ou le cas échéant un ou deux groupes méthylène non-voisins sont remplacés par -O-, -COO- et/ou -OOC- et/ou le cas échéant un groupe méthylène est remplacé par un $-CHY^2-$ ; $Y^1$ représente un hydrogène ou un fluor ; $Y^2$ représente un halogène, cyano, ou méthyle ; $X^1$, $X^2$, $X^3$ et $X^4$ indépendamment l'un de l'autre représentent un hydrogène ou un halogène ; et n, $R^1$, $R^3$, (S*) et (R*) ont les significations données ci-dessus.

**5.** Composés selon la revendication 4, caractérisés en ce que $X^1$, $X^2$, $X^3$ et $X^4$ représentent indépendamment l'un de l'autre un hydrogène, un fluor, ou un chlore.

**6.** Composés selon la revendication 5, caractérisés en ce que $X^1$ représente un hydrogène, un fluor ou un chlore, $X^2$ et $X^3$ représentent un hydrogène et $X^4$ un hydrogène, un fluor ou un chlore.

**7.** Composés selon l'une des revendications 1 à 6, caractérisés en ce que $R^1$ et $R^3$ représentent un alkyle.

**8.** Composés selon l'une des revendications 1 à 7, caractérisés en ce que $R^4$ représente un cyano, fluor, chlore, difluorométhoxy, trifluorométhoxy, difluorométhyle, trifluorométhyle, alkyle, alcoxy, alcoxycarbonyle, 1-(alcoxycarbonyl)-éthoxycarbonyle, alcanoyloxy, 2-fluoroalcanoyloxy, alcényle ou alcényloxy.

**9.** Composés selon l'une des revendications 1 à 8, caractérisés en ce que $R^1$, $R^3$ et $R^4$ présentent 1-18, de préférence 1-12 atomes de carbone.

**10.** Mélange de cristaux liquides avec au moins deux composants, caractérisé en ce qu'au moins un composant est un composé optiquement actif qui est défini par la formule I de la revendication 1.

11. Mélange de cristaux liquides selon la revendication 10, caractérisé en ce qu'il contient un ou plusieurs composés optiquement actifs de formule I et un matériau support de cristaux liquides à phase smectique incliné , cholestérique ou nématique.

12. Mélange de cristaux liquides selon la revendication 10 ou 11, caractérisé en ce que la teneur en composés de formule I représente 0,1-40% en poids.

13. Utilisation de mélanges de cristaux liquides selon la revendication 10 pour des utilisations optiques ou électro-optiques.